# EUROPEAN PATENT SPECIFICATION

(11) **EP 4 220 164 B1**
(45) Date of publication and mention of the grant of the patent: **21.08.2024**
(21) Application number: 23157811.3
(22) Date of filing: 18.12.2017
(51) Int. Cl.: G01N 33/542, G01N 33/543, G01N 33/58, C12Q 1/28

(54) **METHODS AND SYSTEMS FOR QUANTITATIVE IMMUNOHISTOCHEMISTRY**
VERFAHREN UND SYSTEME ZUR QUANTITATIVEN IMMUNHISTOCHEMIE
PROCÉDÉS ET SYSTÈMES D'IMMUNOHISTOCHIMIE QUANTITATIVE

(30) Priority: 19.12.2016 US 201662435955 P
(43) Date of publication of application: 02.08.2023
(62) Divisional of application: 17832420.8
(73) Proprietor: Ventana Medical Systems, Inc., Tucson, Arizona 85755 (US)
(72) Inventor: Day, William, Tucson, AZ 85755 (US); Jiang, Zeyu ( David ), Tucson, AZ 85755 (US); Pedata, Anne, Tucson, AZ 85755 (US)
(74) Representative: Simmons & Simmons LLP (Munich)

(56) References cited:
- WO-A1-2013/148498
- WO-A1-2015/124738
- WO-A1-2016/083364
- WO-A2-2008/036802
- WO-A2-2012/003476
- GEORGE KING ET AL: "A highly sensitive detection method for immunohistochemistry using biotinylated tyramine", THE JOURNAL OF PATHOLOGY, vol. 183, no. 2, 1 October 1997 (1997-10-01), pages 237 - 241, XP055453083, ISSN: 0022-3417, DOI: 10.1002/(SICI)1096-9896(199710)183:2<237::AID-PATH893>3.0.CO;2-0
- DATABASE WPI Week 200854, Derwent World Patents Index; AN 2008-J29610, XP002778533
- DAY WILLIAM A ET AL: "Covalently deposited dyes: a new chromogen paradigm that facilitates analysis of multiple biomarkers in situ", vol. 97, no. 1, 21 November 2016 (2016-11-21), pages 104 - 113, XP055420125, Retrieved from the Internet <URL:http://www.nature.com/articles/labinvest2016115>

## Description

### FIELD OF THE INVENTION

The present invention relates to immunohistochemistry techniques, more particularly to methods and systems for quantitative immunohistochemistry.

### BACKGROUND OF THE INVENTION

Current technologies used in the automated detection of prognostic and/or predictive protein biomarkers rely on a threshold number of biomarker molecules (e.g., greater than one, 1,000, 10,000, etc.) to generate a visible signal. Accordingly, the number of protein biomarker molecules required to generate a visible signal depends on features, primarily sensitivity and specificity, of the detection technology used. Thus, the current technologies do not enable detection or quantification of individual protein molecules. As a result, clinical evaluation of nearly all established prognostic and/or predictive protein biomarkers is limited to the use of binary analysis: plus versus minus, reflecting the presence or absence of signal.

WO2012/003476 discloses detectable labels, which can be used in protein assays.

The present invention features methods of amplifying signals. The present invention helps reduce the threshold number of molecules needed to generate a visible signal. Surprisingly, the methods of the present invention were specific enough to result in punctate dots, as opposed to diffuse signals or blobs, as was expected. The punctate dots can be counted. In certain embodiments, methods of the present invention may be applied to secreted proteins.

The present invention may be used for methods of quantitative immunohistochemistry wherein the punctate dots may represent individual target molecules and can be counted. The present invention enables detection and analysis of secreted protein factors (or other appropriate secreted factors not limited to proteins), whereas current technologies do not generate interpretable signals for proteins secreted from cells. These quantifications are achieved through increases in the amplification systems and chromogens that generate punctate dots signals.

Without wishing to limit the present invention to any theory or mechanism, it is believed that quantification of the signals and expression levels of target molecules may help provide additional prognostic value and/or predictive value for patients as compared to the traditional methods that generate a binary (plus or minus) result.

The present invention also allows for multiplexed signals for multiple target molecules, e.g., using un-mixable dyes with narrow absorbance spectra.

### SUMMARY OF THE INVENTION

Disclosed are methods and systems for quantitative immunohistochemistry. For example, the present invention utilizes a high-sensitivity amplification system (e.g., tyramide-DIG, tyramide-NP) and specific chromogens capable of generating punctate dot signals.

The present invention provides a technology for the detection of individual molecules (e.g., antibodies) bound to individual target protein biomarkers. The generation of punctate dot signals helps enable detection, analysis, and quantification of target protein biomarkers (e.g., proteins or other target molecules, including secreted target protein biomarkers).

'The present invention provides methods for amplifying a signal for a target molecule in a sample (e.g., methods for amplifying a signal for a target molecule in a formalin-fixed paraffin-embedded (FFPE) tissue sample). The method may comprise treating the tissue sample with a deparaffinization reagent and treating the tissue sample with an antigen retrieval reagent. The method may further comprise treating the sample with a protease, e.g., before applying the biomarker-specific agent specific for the target protein biomarker.

The invention provides for a method according to claims 1-14. The method comprises applying to the sample: a biomarker specific agent specific for the target protein biomarkers; a second binding agent specific for the biomarker-specific agent, wherein the second binding agent is conjugated with a secondary enzyme; a tyramide agent comprising a tyramide molecule conjugated with a tyramide hapten, wherein the secondary enzyme catalyzes deposition of the tyramide hapten onto the sample (e.g., at the location of the second binding agent); a third binding agent specific for the tyramide hapten, wherein the third binding agent is conjugated with a tertiary enzyme; and a detectable moiety (e.g., chromogen), wherein the tertiary enzyme catalyzes a reaction with the detectable moiety (e.g., chromogen) to make the detectable moiety (e.g., chromogen) visible. The detectable moiety (e.g., chromogen) may be visible as a punctate dot using microscopy, e.g., brightfield microscopy. The punctate dot may be indicative of the individual target molecule.

In some embodiments, the sample is a tissue sample, e.g., formalin-fixed paraffin-embedded (FFPE) tissue sample; however the sample is not limited to a FFPE tissue sample. Alternative sample compositions are described herein.

In some embodiments, the target biomarker comprises a protein, a carbohydrate, a lipid, a nucleic acid, a post-translational modification (e.g., a phosphate modification, a geranyl modification, an acetyl modification, a ubiquitin modification, a carbohydrate modification, a carbamyl modification, a combination thereof, etc.), the like, or a combination thereof.

The biomarker-specific agent may be an antibody or a fragment thereof, e.g., a primary antibody. In some embodiments, the primary antibody is a native, unmodified antibody. In some embodiments, the primary antibody is monoclonal or polyclonal. The second binding agent may be an antibody or fragment thereof, e.g., a secondary antibody. In some embodiments, the secondary antibody comprises an anti-species antibody, an anti-modification antibody, or a combination thereof. In some embodiments, the secondary antibody enzyme comprises an oxidoreductase, a hydrolase, or a peroxidase, e.g., horseradish peroxidase (HRP).

Examples of tyramide haptens are described herein. In some embodiments, the tyramide hapten comprises biotin, digoxigenin (DIG), nitropyrazole (NP), benzofurazan (BF), benzodazapine (BD), nitrocinnamide (NCA), fluorescein, dinitrophenyl (DNP), the like, etc.

The third binding agent may be an antibody or fragment thereof. In some embodiments, the third binding agent comprises a tertiary antibody, e.g., a monoclonal antibody.

In some embodiments, the detectable moiety comprises silver or a tyramide-rhodamine dye (e.g., rhodamine 110, rhodamine 6G, tetramethylrhodamine (TAMRA), sulforhodamine B, sulforhodamine 101 (Texas Red), or a combination thereof, etc.), DAB, 4-nitrophenylphospate (pNPP), fast red, bromochloroindolyl phosphate (BCIP), nitro blue tetrazolium (NBT), BCIP/NBT, fast red, AP Orange, AP blue, tetramethylbenzidine (TMB), 2,2'-azino-di-[3-ethylbenzothiazoline sulphonate] (ABTS), o -dianisidine, 4-chloronaphthol (4-CN), nitrophenyl-β-D-galactopyranoside (ONPG), o-phenylenediamine (OPD), 5-bromo-4-chloro-3-indolyl-β-galactopyranoside (X-Gal), methylumbelliferyl-β-D-galactopyranoside (MU-Gal), p-nitrophenyl-α-D-galactopyranoside (PNP), 5-bromo-4-chloro-3-indolyl- β -D-glucuronide (X-Gluc), 3-amino-9-ethyl carbazol (AEC), fuchsin, iodonitrotetrazolium (INT), tetrazolium blue, or tetrazolium violet.

The methods of the present invention may be applied to a multiplex immunohistochemistry (IHC) assay wherein two or more target molecules can be detected and distinguished, e.g., one or more target biomarkers, one or more target biomarkers and one or more secreted target biomarkers, etc.

The present invention also features an automated staining machine comprising a system adapted to perform a method of the present invention. The automated staining machine may comprise a memory coupled to a processor, wherein the memory stores computer-readable instructions that, when executed by the processor, cause the automated staining machine to perform operations for a method of the present invention. The present invention also features an automated system comprising a slide holder, immunohistochemistry reagents, and dispensers for performing a method of the present invention. For example, the dispensers may be adapted to dispense immunohistochemistry reagents onto a slide in the slide holder.

Any feature or combination of features described herein are included within the scope of the present invention provided that the features included in any such combination are not mutually inconsistent as will be apparent from the context, this specification, and the knowledge of one of ordinary skill in the art. Additional advantages and aspects of the present invention are apparent in the following detailed description and claims.

### BRIEF DESCRIPTION OF THE DRAWINGS

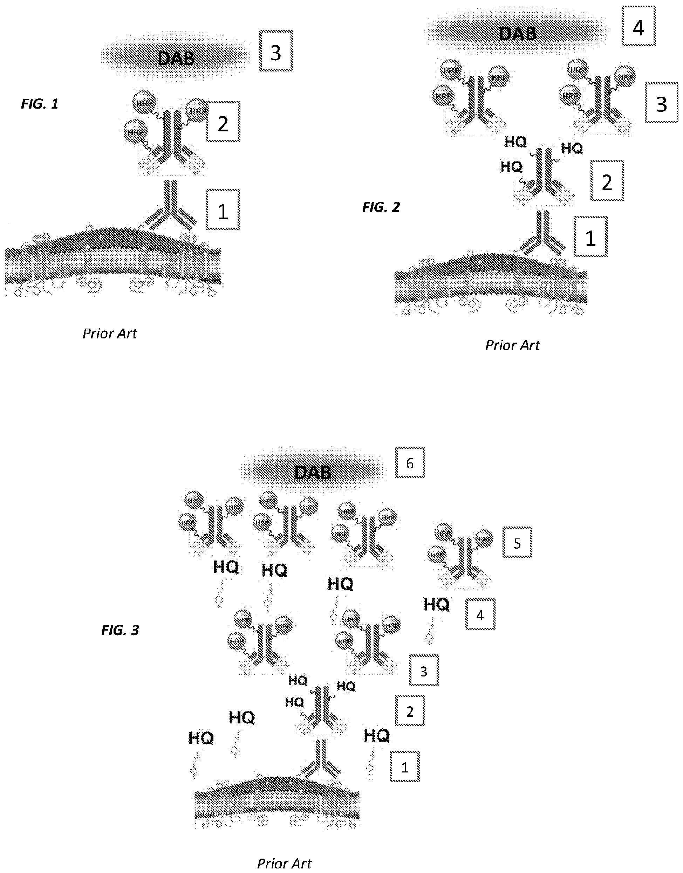

FIG. 1 (prior art) shows a schematic view of an example of routine detection technology. (1) A primary antibody (native, unmodified antibody) binds a protein target in tissue. (2) A secondary antibody (a mixture of goat-anti-mouse and goat-anti-rabbit polyclonal antibodies conjugated to HRP enzyme) is added. (3) DAB chromogen is added.
FIG. 2 (prior art) shows a schematic view of an example of a detection method. (1) A primary antibody (native, unmodified antibody) binds a protein target in tissue. (2) A secondary antibody (a mixture of goat-anti-mouse and goat-anti-rabbit polyclonal antibodies conjugated to HQ hapten) is added. (3) A tertiary antibody (anti-HQ monoclonal antibody conjugated to HRP enzyme) is added. (4) DAB chromogen is added.
FIG. 3 (prior art) shows a schematic view of an example of a detection method. (1) A primary antibody (native, unmodified antibody) binds a protein target in tissue. (2) A secondary antibody (a mixture of goat-anti-mouse and goat-anti-rabbit polyclonal antibodies conjugated to HQ hapten) is added. (3) A tertiary antibody (anti-HQ monoclonal antibody conjugated to HRP enzyme) is added. (4) Tyramide-HQ hapten conjugate is added (hapten amplification step). (5) A quaternary antibody (anti-HQ monoclonal antibody conjugated to HRP enzyme) is added. (6) DAB chromogen is added.

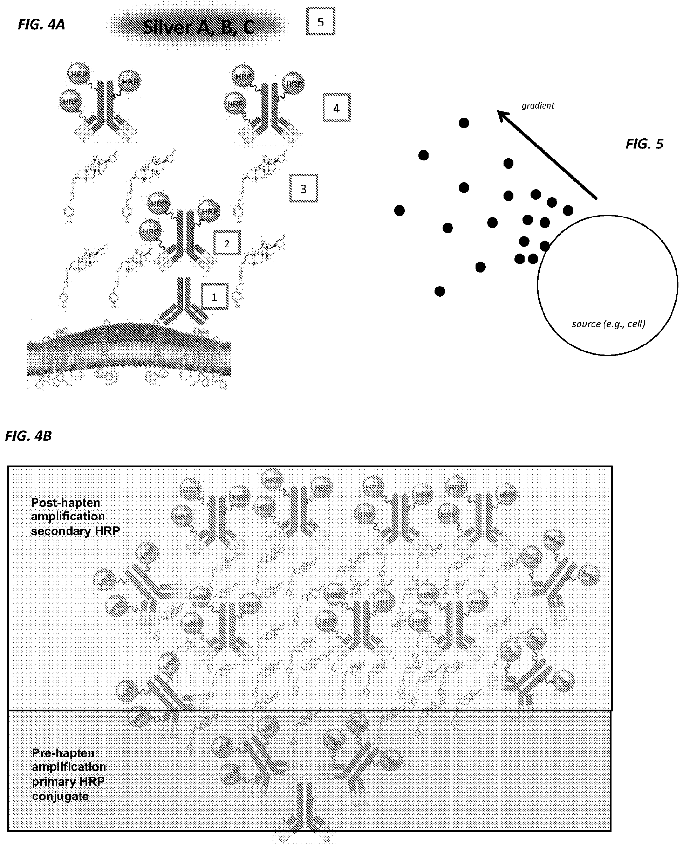

FIG. 4A shows a schematic view of an example of a detection method of the present invention. (1) A primary antibody (native, unmodified antibody) binds a protein target in tissue. (2) A secondary antibody (anti-species polyclonal antibodies, or anti-modification antibody, etc., conjugated to HRP enzyme) is added. (3) Tyramide (DIG, NP, BF, NCA, BD, or DNP, etc. hapten conjugate) is added (hapten amplification step). (4) A tertiary antibody (anti-hapten monoclonal antibody conjugated to HRP enzyme) is added. (5) Silver or tyramide-rhodamine dye chromogen is added. Note that the present invention is not limited to the aforementioned steps or reagents.
FIG. 4B shows a schematic view of methods described herein. A native or modified (e.g., haptenated, tagged) antibody (e.g., monoclonal antibody) binds to the target biomarker. Either a monoclonal or polyclonal secondary antibody conjugate (e.g., HRP) is used to bind to the target-specific binding agent (e.g., the primary antibody). Tyramide conjugate is added. Then an antibody (e.g., a monoclonal antibody (e.g., monoclonal HRP antibody)) binds to the tyramide conjugate. Without wishing to limit the present invention to any theory or mechanism, it is believed that fewer primary HRP conjugates (e.g., secondary antibodies) bound to the primary antibody necessitates a higher hapten amplification (e.g., as compared to RNA probe detection) for sufficient secondary HRP deposition to drive visible chromogen signal.
FIG. 5 shows a schematic view of a gradient of secreted proteins secreted from a source (e.g., cell).

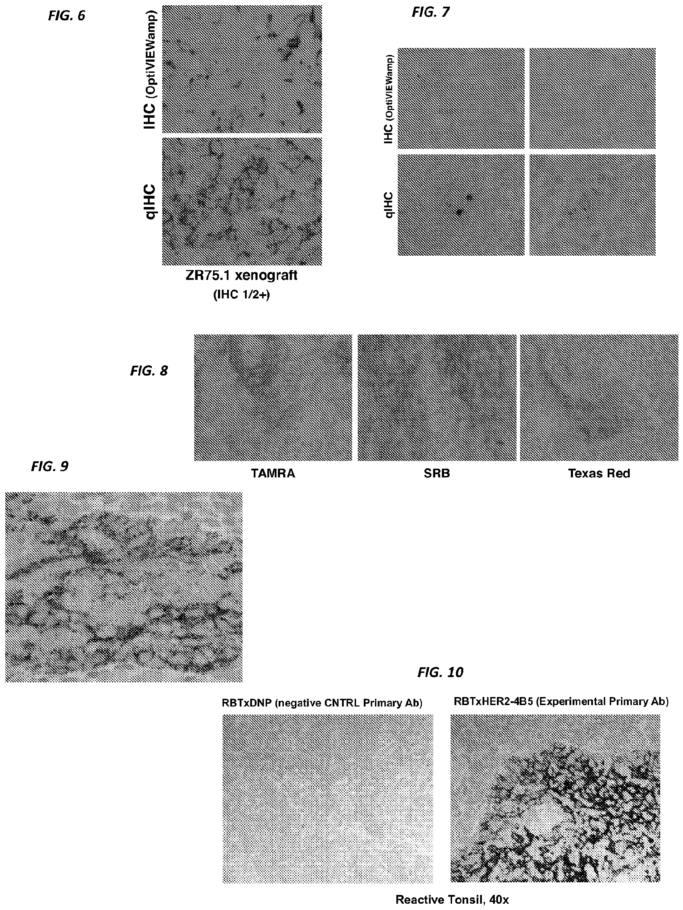

FIG. 6 shows an example of immunohistochemistry (IHC) of HER2 in a ZR75.1 xenograft using traditional methods of IHC (top panel) and methods of the present invention, qIHC (bottom panel). Note the punctate dot signals observed at low to medium expression levels of HER2 using the methods of the present invention (qIHC) compared to the diffuse staining using traditional methods.
FIG. 7 shows an example of immunohistochemistry (IHC) of Interferon gamma (IFN gamma), a secreted protein factor, in reactive tonsil using traditional methods of IHC (top panels) and methods of the present invention, qIHC (bottom panels). Note the punctate dot signals of IFN gamma observed using the methods of the present invention (qIHC) compared to the diffuse staining using traditional methods. This suggests the methods of the present invention may be used for secreted target molecules.
FIG. 8 shows examples of punctate IHC signals achieved with tyramide-chromogens used in methods of the present invention.
FIG. 9 shows multiplex detection of Her2 protein in tonsil tissue showing detection of protein molecules, e.g., single protein molecules.
FIG. 10 shows detection of Her2 protein in tonsil tissue demonstrating specificity of the assay. The left panel shows the control sample and the right panel shows the sample stained for Her2.

### TERMS

Unless otherwise explained, all technical and scientific terms used herein have the same meaning as commonly understood by one of ordinary skill in the art to which a disclosed invention belongs. The singular terms "a," "an," and "the" include plural referents unless context clearly indicates otherwise. Similarly, the word "or" is intended to include "and" unless the context clearly indicates otherwise. "Comprising" means "including." Hence "comprising A or B" means "including A" or "including B" or "including A and B."

Suitable methods and materials for the practice and/or testing of embodiments of the disclosure are described below. Such methods and materials are illustrative only and are not intended to be limiting. Other methods and materials similar or equivalent to those described herein can be used. For example, conventional methods well known in the art to which the disclosure pertains are described in various general and more specific references, including, for example, Sambrook et al., Molecular Cloning: A Laboratory Manual, 2d ed., Cold Spring Harbor Laboratory Press, 1989; Sambrook et al., Molecular Cloning: A Laboratory Manual, 3d ed., Cold Spring Harbor Press, 2001; Ausubel et al., Current Protocols in Molecular Biology, Greene Publishing Associates, 1992 (and Supplements to 2000); Ausubel et al., Short Protocols in Molecular Biology: A Compendium of Methods from Current Protocols in Molecular Biology, 4th ed., Wiley & Sons, 1999; Harlow and Lane, Antibodies: A Laboratory Manual, Cold Spring Harbor Laboratory Press, 1990; and Harlow and Lane, Using Antibodies: A Laboratory Manual, Cold Spring Harbor Laboratory Press, 1 999.

Although methods and materials similar or equivalent to those described herein can be used to practice or test the disclosed technology, suitable methods and materials are described below. The materials, methods, and examples are illustrative only and not intended to be limiting.

In order to facilitate review of the various embodiments of the disclosure, the following explanations of specific terms are provided:
**Antibody:** A polypeptide that includes at least a light chain or heavy chain immunoglobulin variable region and specifically binds an epitope of an antigen (such as HER2 protein or ER protein). Antibodies include monoclonal antibodies, polyclonal antibodies, or fragments of antibodies. An antibody can be conjugated or otherwise labeled with a detectable label, such as an enzyme, hapten, etc.
**Antibody fragment:** A molecule other than an intact antibody that comprises a portion of an intact antibody that binds the antigen to which the intact antibody binds. Examples of antibody fragments include but are not limited to Fv, Fab, Fab', Fab'-SH, F(ab')2; diabodies; linear antibodies; single-chain antibody molecules (e.g. scFv); and multispecific antibodies formed from antibody fragments.
**Biological sample:** As used herein, the term "biological sample" shall refer to any material obtained from a subject capable of being tested for the presence or absence of a biomarker or target molecule.
**Biomarker or Target Molecule:** As used herein, the term "biomarker" or "target molecule" shall refer to any molecule or group of molecules found in a biological sample that can be used to characterize the biological sample or a subject from which the biological sample is obtained. For example, a biomarker may be a molecule or group of molecules whose presence, absence, or relative abundance is: characteristic of a particular disease state; indicative of the severity of a disease or the likelihood or disease progression or regression; and/or predictive that a pathological condition will respond to a particular treatment. As another example, the biomarker may be an infectious agent (such as a bacterium, fungus, virus, or other microorganism), or a substituent molecule or group of molecules thereof.
**Biomarker-specific agent:** Any compound or composition that binds to a biomarker or a specific structure within that biomarker in a manner that permits a specific detection of the biomarker in a sample. Examples include: antibodies and antigen binding fragments thereof; and engineered specific binding structures, including ADNECTINs (scaffold based on 10th FN3 fibronectin; Bristol-Myers-Squibb Co.), AFFIBODYs (scaffold based on Z domain of protein A from S. aureus; Affibody AB, Solna, Sweden), AVIMERs (scaffold based on domain A/LDL receptor; Amgen, Thousand Oaks, CA), dAbs (scaffold based on VH or VL antibody domain; GlaxoSmithKline PLC, Cambridge, UK), DARPins (scaffold based on Ankyrin repeat proteins; Molecular Partners AG, Zürich, CH), ANTICALINs (scaffold based on lipocalins; Pieris AG, Freising, DE), NANOBODYs (scaffold based on VHH (camelid Ig); Ablynx N/V, Ghent, BE), TRANS-BODYs (scaffold based on Transferrin; Pfizer Inc., New York, NY), SMIPs (Emergent Biosolutions, Inc., Rockville, MD), and TETRANECTINs (scaffold based on C-type lectin domain (CTLD), tetranectin; Borean Pharma A/S, Aarhus, DK) (Descriptions of such engineered specific binding structures are reviewed by Wurch et al., Development of Novel Protein Scaffolds as Alternatives to Whole Antibodies for Imaging and Therapy: Status on Discovery Research and Clinical Validation, Current Pharmaceutical Biotechnology, Vol. 9, pp. 502-509 (2008); and fusion proteins including at least a first domain capable of specifically binding to the biomarker (e.g. an antigen binding fragment of an antibody or a target-binding portion of a protein that binds to the biomarker) and a second portion that is adapted to facilitate binding of detection reagents to the fusion protein (e.g., a biotin label, an epitope tag, an Ig fragment, etc.).
**Cellular sample:** As used herein, the term "cellular sample" refers to any sample containing intact cells, such as cell cultures, bodily fluid samples or surgical specimens taken for pathological, histological, or cytological interpretation.
**Conjugate:** to two or more molecules that are covalently linked into a larger construct. In some embodiments, a conjugate includes one or more biomolecules (such as peptides, nucleic acids, proteins, enzymes, sugars, polysaccharides, lipids, glycoproteins, and lipoproteins) covalently linked to one or more other molecules, such as one or more other biomolecules. In other embodiments, a conjugate includes one or more specific-binding molecules (such as antibodies and nucleic acid sequences) covalently linked to one or more detectable labels (haptens, enzymes and combinations thereof). In other embodiments, a conjugate includes one or more latent reactive moieties covalently linked to detectable labels (haptens, chromophore moieties).
**Contacting:** placement that allows association between two or more moieties, particularly direct physical association, for example both in solid form and/or in liquid form (for example, the placement of a biological sample, such as a biological sample affixed to a slide, in contact with a composition, such as a solution containing the probes disclosed herein).
**Detectable label or Detectable moiety:** A molecule or material that can produce a detectable signal (such as a visual, electrical, or other signal) that indicates the presence and/or concentration of the detectable moiety or label deposited on the sample (or the presence and/or amount of a target (such as a protein or nucleic acid) in a sample). Detectable labels are well known to one of ordinary skill in the art.

A detectable signal can be generated by any known or yet to be discovered mechanism including absorption, emission and/or scattering of a photon (including radio frequency, microwave frequency, infrared frequency, visible frequency and ultra-violet frequency photons).

When conjugated to a specific binding molecule (for example, an antibody or nucleic acid probe), the detectable label can be used to locate and/or quantify the target to which the specific binding molecule is directed. A detectable label can be detected directly or indirectly, and several different detectable labels can be used in combination to detect one or more targets. For example, a first detectable label, such as a hapten conjugated to an antibody specific to a target, can be detected indirectly by using a second detectable label that is conjugated to a molecule that specifically binds the first detectable label. In addition, multiple detectable labels that can be separately detected can be conjugated to different specific binding molecules that specifically bind different targets to provide a multiplex assay that can provide detection of the multiple targets in a single sample.

Detectable labels or detectable moieties include but are not limited to chromogenic, phosphorescent and/or luminescent molecules, catalysts (such as enzymes) that convert one substance into another substance to provide a detectable signal (such as by converting a colorless substance into a colored substance or vice versa, or by producing a precipitate or increasing sample turbidity), haptens that can be detected through antibody-hapten binding interactions using additional detectably labeled antibody conjugates, and paramagnetic and magnetic molecules or materials. Particular examples of detectable labels include: enzymes, such as horseradish peroxidase, alkaline phosphatase, acid phosphatase, glucose oxidase, β-galactosidase or β-glucuronidase; nanoparticles, such as quantum dots (U.S. Patent Nos. 6,815,064, 6,682596 and 6,649,138, metal chelates, such as DOTA and DPTA chelates of radioactive or paramagnetic metal ions like Gd³⁺; and liposomes, for example, liposomes containing trapped molecules. Where the detectable label includes an enzyme, a detectable substrate such as a chromogen, or a luminogenic compound is used in combination with the enzyme to generate a detectable signal (a wide variety of such compounds are commercially available, for example, from Life Technologies, Carlsbad, CA).

In other embodiments, the detectable moiety is a molecule detectable via brightfield microscopy, such as dyes including diaminobenzidine (DAB), 4-(dimethylamino) azobenzene-4'-sulfonamide (DABSYL), tetramethylrhodamine (DISCOVERY Purple), N,N'-biscarboxypentyl-5,5'-disulfonato-indo-dicarbocyanine (Cy5), and Rhodamine 110 (Rhodamine).

Alternatively, an enzyme can be used in a metallographic detection scheme. In some examples, metallographic detection methods include using an enzyme, such as alkaline phosphatase, in combination with a water-soluble metal ion and a redox-inactive substrate of the enzyme. The substrate is converted to a redox-active agent by the enzyme, and the redox-active agent reduces the metal ion, causing it to form a detectable precipitate (see, for example, U.S. Pat. Nos. 7,642,064, 7,632,652. In other examples, metallographic detection methods include using an oxido-reductase enzyme (such as horseradish peroxidase) along with a water soluble metal ion, an oxidizing agent and a reducing agent, again to form a detectable precipitate (see, for example, U.S. Patent No. 6,670,1 13. Haptens are small molecules that can be bound by antibodies. Exemplary haptens include dinitrophenyl (DNP), biotin, digoxigenin (DIG), and fluorescein. Additional haptens include oxazole, pyrazole, thiazole, nitroaryl, benzofuran, triperpene, urea, thiourea, rotenoid, coumarin and cyclolignan haptens, such as those disclosed in U.S. Pat. No. 7,695,929.

**Detection reagent:** When used in connection with a histochemical assay (including immunohistochemistry and affinity histochemistry), any reagent that is used to deposit a stain in proximity to a biomarker-specific agent bound to a biomarker in a cellular sample. Non-limiting examples include secondary antibodies capable of binding to a biomarker-specific antibody; enzymes linked to such secondary antibodies; and chemicals reactive with such enzymes to effect deposition of a chromogenic stain; and the like.

**Hapten:** A hapten is a molecule, typically a small molecule, which can combine specifically with an antibody, but typically is substantially incapable of being immunogenic except in combination with a carrier molecule. Many haptens are known and frequently used for analytical procedures, such as dinitrophenyl, biotin, digoxigenin, fluorescein, rhodamine, or those disclosed in U.S. Pat. No. 7,695,929. haptens have been specifically developed by Ventana Medical Systems, Inc., assignee of the present application, including haptens selected from oxazoles, pyrazoles, thiazoles, nitroaryls, benzofurans, triterpenes, ureas, thioureas, rotenoids, coumarins, cyclolignans, and combinations thereof, with particular hapten examples of haptens including benzofurazan, nitrophenyl, 4-(2-hydroxyphenyl)-1H-benzo[b][1,4]diazepine-2(3H)-one, and 3-hydroxy-2-quinoxalinecarbamide. Plural different haptens may be coupled to a polymeric carrier. Moreover, compounds, such as haptens, can be coupled to another molecule using a linker, such as an NHS-PEG linker.

**Histochemical detection:** A process involving labeling a biomarker or other structures in a tissue sample with detection reagents in a manner that permits microscopic detection of the biomarker or other structures in the context of the cross-sectional relationship between the structures of the tissue sample. Examples include but are not limited to affinity histochemistry (AHC), immunohistochemistry (IHC), chromogenic *in situ* hybridization (CISH), silver *in situ* hybridization (SISH), and hematoxylin and eosin (H&E) staining of formalin-fixed, paraffin-embedded tissue sections.

**Histochemistry (e.g., see Immunohistochemistry (IHC) also):** A method of determining the presence or distribution of a target molecule in a sample by detecting interaction of the target molecule with a specific binding agent, such as an antibody, that can be detected. For example, a sample is contacted with an antibody (or other binding agent such as an antibody fragment, etc.) under conditions permitting antibody-antigen binding. Antibody-antigen binding can be detected by means of a detectable label conjugated to the antibody (direct detection) or by means of a detectable label conjugated to a secondary antibody, which binds specifically to the primary antibody (*e*.*g*., indirect detection).

**Immunohistochemistry (IHC):** A technique that utilizes antibodies or derivatives thereof or other proteinaceous binding agents to analyze histological tissues under the microscope. Due to the inherent nature of different types of histological tissues composing the body as well as the complexity of tissue antigens, there are no universal "one-size-fits-all" staining protocols in IHC. Generally, a workflow of IHC staining may be as follows: a. Hydrophobic tissue protection: a hydrophobic barrier line is used around the tissue section to prevent leakage of reagents from the slide during incubation; b. Blocking: Tissue sections are treated with reagents to block endogenous sources of nonspecific staining such as (i) enzymes, (ii) endogenous peroxidase, (iii) free aldehyde groups, (iv) immunoglobulins, and other irrelevant molecules that can mimic specific staining; c. Permeabilization (this step may be used as needed): Tissue sections are incubated with permeabilization buffer to facilitate penetration of antibodies and other staining reagents into the tissue; d. Incubation with primary (often depicted as 1°Ab) antibody: This incubation may be done for several hours (e.g., 1-24 hours) either at room temperature or in a cold room (e.g., at 6-8 °C) depending on the affinity of antibodies and abundance of tissue target; e. Rinsing with wash buffer: This step may be done as short, repetitive cycles (e.g., 3 - 5 cycles of 5-15 min) using fresh wash buffer; f. Incubation with secondary (may be depicted as 2°Ab) antibody: This may be done for several hours (e.g., 1-2 hours) at room temperature; g. Rinsing with wash buffer: This is a repeat of step "e"; and h. Incubations with detection reagents, mingled with rinsing with wash buffer: This may be done when applicable (for example, in chromogenic detection). The present invention is not limited to this IHC protocol.

Counterstaining is the staining of tissue sections with dyes that allow one to see the entire "landscape" of the tissue section and serve as a reference for the main color used for the detection of tissue targets. Such dyes can stain cell nuclei, the cell membrane, or the entire cell. Examples of dyes include DAPI, which binds to nuclear DNA and emits strong blue light; Hoechst blue stain, which binds to nuclear DNA and emits strong blue light; and Propidium iodide, which binds to nuclear DNA and emits strong red light. Counterstaining of the intracellular cytoskeletal network can be done using phalloidin conjugated to dyes. Phalloidin is a toxin that tightly binds to actin filaments in a cell's cytoplasm, which then become clearly visible under the microscope.

The majority of chromogenic IHC protocols are based on the use of Avidin and Biotin molecules because detection sensitivity of a simple antigen-antibody reaction in many cases is quite low. Avidin-Biotin binding serves to bridge antigen-bound antibodies with detection reagents, allowing amplification of the staining signal. The most frequently used Biotin-based techniques include labeled SA-Biotin (LSAB) and Avidin-Biotin Complex (ABC) detection. There are also non-Biotin-based detection techniques utilizing primary antibodies either conjugated directly to enzymatic labels or to a long polymer containing multiple copies of enzymatic labels.

LSAB Detection utilizes secondary antibodies conjugated to Biotin that link primary antigen-bound antibodies to SA conjugated to an enzyme. The first step in LSAB detection is the incubation of tissue sections with primary antibodies followed by incubation with biotinylated secondary antibodies. After that, SA conjugated to the enzyme of choice (e.g., AP, HRP, etc.) is added to tissue sections followed by adding appropriate enzyme substrate. The enzyme converts substrate into colored particles precipitating at the sites of antigen localization, which can then be observed under the microscope. LSAB technique can be shortened using biotinylated primary antibodies, eliminating the need for incubation with biotinylated secondary antibodies.

The initial steps-incubation with primary and biotinylated secondary antibodies-in ABC detection are the same as in LSAB, but the next steps and reagents are quite different. Avidin and biotinylated enzymes are first mixed and incubated together for about 30 min at room temperature and then added to tissue sections. During this incubation, Avidin interacts with the biotinylated enzymes, forming large complexes densely packed with enzyme molecules-far exceeding the concentration found in the LSAB detection technique-that boost the sensitivity of antigen detection.

Non-Biotin detection techniques have gained popularity because they are devoid of such limitations of Avidin-Biotin detection as nonspecific background staining due to the endogenous biotin that is abundant in different types of animal tissues, including kidney, brain, and placenta.

In chromogenic IHC, tissue counterstaining serves to visualize the entire layout of the tissue section and label organelles of the same type. Usually counterstaining is done to label cell nuclei that should not be of the same color as the main color depicting antigens of interest. For example, if the main color is red (AEC chromogen) or brown (DAB chromogen), nuclei may be stained using Hematoxylin, which produces a blue color, or Methyl Green, which produces a green color. If the main color is blue (BCIP/NBT chromogen), then nuclei may be counterstained red using Nuclear Fast Red dye. In cases when tissue antigen is localized in cell nuclei, the duration of their counterstaining may be either shortened to make them barely visible or even skipped to avoid masking the main IHC color.

AHC refers to affinity histochemistry wherein detection of a biomarker involves the use of a binding agent with affinity for the biomarker. For example, mast cells may be stained by AHC based on electrostatic attractions between the basic protein avidin and the polyanionic heparin.

**Multiplex, -ed, -ing:** Embodiments of the present invention allow multiple targets in the same sample to be detected, e.g., substantially simultaneously, or sequentially, as desired.

**Punctate dot:** As used herein, the term "punctate dot" refers to a dot-like appearance of staining, wherein the dot is distinguishable from diffuse staining. Diffuse signal patters (e.g., typical DAB signal patterns) do not generally generate signals that can be resolved individually. The punctate dots can also be resolved from each other (e.g., counted). For example, FIG. 6 shows punctate dot signals observed at low to medium expression levels of HER2 (bottom pane) using the methods of the present invention (qIHC) compared to the diffuse staining using traditional methods (top panel). The punctate dots are distinct from diffuse staining, since diffuse staining covers a larger area of the tissue sample. Further, punctate dots can be counted individually.

**Sample and Biological Sample:** Any composition containing or presumed to contain a biomarker or a composition being tested for the presence or absence of a particular biomarker. Samples may include purified or separated components of cells, tissues, or blood, e.g., DNA, RNA, proteins, cell-free portions, or cell lysates. The sample can be a formalin-fixed, paraffin-embedded (FFPE) tissue sample, e.g., from a tumor or metastatic lesion, e.g., primary tumor or metastatic tumor. The sample can also be from previously frozen or fresh tissue, or from a liquid sample, e.g., blood or a blood component (plasma or serum), urine, semen, saliva, sputum, mucus, semen, tear, lymph, cerebral spinal fluid, material washed from a swab, etc. Samples also may include constituents and components of in vitro cultures of cells obtained from an individual, including cell lines. The sample can also be partially processed from a sample directly obtained from an individual, e.g., cell lysate or blood depleted of red blood cells.

**Section:** When used as a noun, a thin slice of a tissue sample suitable for microscopic analysis, typically cut using a microtome. When used as a verb, making a section of a tissue sample, typically using a microtome.

**Serial Section:** Any one of a series of sections cut in sequence from a tissue sample. For two sections to be considered "serial sections" of one another, they do not necessarily need to consecutive sections from the tissue, but they should generally contain the same tissue structures in the same cross-sectional relationship, such that the structures can be matched to one another after histological staining.

**Specific Binding:** As used herein, the phrase "specific binding," "specifically binds to," or "specific for" refers to measurable and reproducible interactions such as binding between a target and a specific binding agent, which is determinative of the presence of the target in the presence of a heterogeneous population of molecules including biological molecules. For example, a binding entity that specifically binds to a target is an antibody that binds this target with greater affinity, avidity, more readily, and/or with greater duration than it binds to other targets. In one embodiment, the extent of binding of a binding entity to an unrelated target is less than about 10% of the binding of the antibody to the target as measured, e.g., by a radioimmunoassay (RIA). In certain embodiments, a binding entity that specifically binds to a target has a dissociation constant (Kd) of ≤1 µM, ≤100 nM, ≤10 nM, ≤1 nM, or ≤0.1 nM. In another embodiment, specific binding can include, but does not require exclusive binding.

**Specific binding agent:** As used herein, the term "specific binding agent" shall refer to any compound or composition that is capable of specifically binding to a biomarker or a specific structure within that biomarker. Examples include but are not limited to nucleic acid probes specific for particular nucleotide sequences; antibodies and antigen binding fragments thereof; and engineered specific binding structures, including ADNECTINs (scaffold based on 10th FN3 fibronectin; Bristol-Myers-Squibb Co.), AFFIBODYs (scaffold based on Z domain of protein A from S. aureus; Affibody AB, Solna, Sweden), AVIMERs (scaffold based on domain A/LDL receptor; Amgen, Thousand Oaks, CA), dAbs (scaffold based on VH or VL antibody domain; GlaxoSmithKline PLC, Cambridge, UK), DARPins (scaffold based on Ankyrin repeat proteins; Molecular Partners AG, Zürich, CH), ANTICALINs (scaffold based on lipocalins; Pieris AG, Freising, DE), NANOBODYs (scaffold based on VHH (camelid Ig); Ablynx N/V, Ghent, BE), TRANS-BODYs (scaffold based on Transferrin; Pfizer Inc., New York, NY), SMIPs (Emergent Biosolutions, Inc., Rockville, MD), and TETRANECTINs (scaffold based on C-type lectin domain (CTLD), tetranectin; Borean Pharma A/S, Aarhus, DK). Descriptions of such engineered specific binding structures are reviewed by Wurch et al., Development of Novel Protein Scaffolds as Alternatives to Whole Antibodies for Imaging and Therapy: Status on Discovery Research and Clinical Validation, Current Pharmaceutical Biotechnology, Vol. 9, pp. 502-509 (2008).

**Stain:** When used as a noun, the term "stain" shall refer to any substance that can be used to visualize specific molecules or structures in a cellular sample for microscopic analysis, including brightfield microscopy, electron microscopy, and the like. When used as a verb, the term "stain" shall refer to any process that results in deposition of a stain on a cellular sample.

**Subject:** Any multi-cellular vertebrate organism, such as human or non-human mammals (*e*.*g*., veterinary subjects).

**Tissue sample:** As used herein, the term "tissue sample" shall refer to a cellular sample that preserves the cross-sectional spatial relationship between the cells as they existed within the subject from which the sample was obtained. "Tissue sample" shall encompass both primary tissue samples (i.e. cells and tissues produced by the subject) and xenografts (i.e. foreign cellular samples implanted into a subject).

### DETAILED DESCRIPTION OF THE INVENTION

Examples of current IHC technologies are shown in FIG. 1, FIG. 2, and FIG. 3. The example method shown in FIG. 1 features a primary antibody binding to the target and a secondary antibody with HRP binding to the primary antibody. DAB chromogen is used. The example method shown in FIG. 2 features a primary antibody binding to the target and a secondary antibody with HQ hapten binding to the primary antibody. A tertiary antibody with HRP binds to the secondary antibody. DAB chromogen is used. The example method shown in FIG. 3 features a primary antibody binding to the target and a secondary antibody with HQ hapten binding to the primary antibody. A tertiary antibody with HRP binds to the secondary antibody. A tyramide-HQ hapten conjugate is added, and a quaternary antibody with HRP enzyme is added. DAB chromogen is used. These current technologies involve detection schemes that yield generally diffuse staining, as an example with DAB.

In certain embodiments, the present invention features methods and systems for quantitative immunohistochemistry (qIHC), allowing for quantifying one or more target molecules. For example, the methods herein produce visible punctate dots (that in certain embodiments allow for the counting of individual molecules (e.g., target molecules)), e.g., by reducing the threshold number of molecules needed to generate a visible signal. For example, the present invention utilizes a high-sensitivity amplification system and chromogens that generate punctate dots signals. In certain embodiments, the present invention provides a technology for the detection of individual antibodies bound to individual target molecules. For example, the example method shown in FIG. 4A features a primary antibody binding to the target and a secondary antibody with HRP binding to the primary antibody. Tyramide-hapten conjugate (e.g., DIG, NP, BF, NCA, BD, or DNP, etc. hapten conjugate) is added as well as a tertiary antibody with HRP. Lastly, silver or tyramide-rhodamine dye chromogen is added (or other appropriate chromogen is added). FIG. 4B shows a native or modified (e.g., haptenated, tagged) antibody (e.g., monoclonal antibody) binding to a target biomarker. Either a monoclonal or polyclonal secondary antibody conjugate (e.g., HRP) is used to bind to the target-specific binding agent (e.g., the primary antibody). Tyramide conjugate is added. Then an antibody (e.g., a monoclonal antibody (e.g., monoclonal HRP antibody)) binds to the tyramide conjugate. Without wishing to limit the present invention to any theory or mechanism, it is believed that fewer primary HRP conjugates (e.g., secondary antibodies) bound to the primary antibody necessitates a higher hapten amplification (e.g., as compared to RNA probe detection) for sufficient secondary HRP deposition to drive visible chromogen signal. The present invention is not limited to the aforementioned steps or reagents. The present invention is not limited to single molecule detection.

The present invention also includes quantitative immunohistochemistry (qIHC) of secreted target molecules (e.g., secreted proteins, etc.) and quantitative immunohistochemistry (qIHC) for detecting multiple target molecules, e.g., using un-mixable dyes. FIG. 5 shows an example of secreted factors secreted from a source (e.g., a cell). Current technologies do not generate interpretable signals for proteins secreted from cells. In some embodiments, the methods herein increase the amplification of a signal (resulting in the targets appearing as visible punctate dots), and this amplification of signal allows for the detection of secreted factors. In certain embodiments, the methods of the present invention detect secreted factors such as secreted proteins (or other appropriate secreted factors not limited to proteins) (see also FIG. 7). In certain embodiments, the methods of the present invention detect a pattern of diffusion and/or a gradient of secreted factors (e.g., a pattern of diffusion and/or a gradient away from a source, e.g., a cell).

The methods of the present invention may be applied to samples such as but not limited to tissue samples (e.g., tissue samples such as FFPE tissue samples of from a patient or other subject).

The target molecule detected may be any appropriate target molecule such as a protein, lipid, carbohydrate, a post-translational modifications (including but not limited to a phosphate modification, a geranyl modification, an acetyl modification, a ubiquitin modification, a carbohydrate modification, a carbamyl modification, the like, etc.), the like, combinations thereof, etc., including secreted target molecules.

### Samples and Sample Preparation

The methods of the present invention are modeled herein on tissue sections adhered to slides. The tissue sections may comprise healthy tissue, diseased tissue, or a combination thereof. The tissue sections may be derived from any appropriate tissue, e.g., skin, breast, head and/or neck, lung, upper gastrointestinal tract (e.g., the esophagus and stomach), female reproductive system (e.g., uterine, fallopian tubes, and ovary), male reproductive system (e.g., prostate, testicles, etc.), lower gastrointestinal tract (e.g., colon, rectal, and anus), urogenital tract, exocrine, endocrine, renal, neural, a lymphocytic origin, vascular tissue, cardiac tissue, nervous system tissue, blood, bone, the like, or a combination thereof.

In some embodiments, the tissue sections comprise a tumor or metastatic lesion. The tumor may be a solid tumor, such as a carcinoma, lymphoma, or sarcoma. In some embodiments, the tumor is a tumor of the skin, breast, head and/or neck, lung, upper gastrointestinal tract (including the esophagus and stomach), female reproductive system (including uterine, fallopian, and ovarian tumors), lower gastrointestinal tract (including the colon, rectal, and anal tumors), urogenital tract, exocrine, endocrine, renal, neural, or of lymphocytic origin. In some embodiments, the tissue section is derived from a subject that has a melanoma, breast cancer, ovarian cancer, pancreatic cancer, head and neck cancer, lung cancer, esophageal cancer, gastric cancer, colorectal cancer (including cancer of the colon, rectum, and anus), prostate, urothelial cancer, or lymphoma.

The sample may be from previously frozen or fresh tissue, or from a liquid sample, e.g., blood or a blood component (plasma or serum), urine, semen, saliva, sputum, mucus, semen, tear, lymph, cerebral spinal fluid, material washed from a swab, etc. In some embodiments, the sample comprises *in vitro* cultures of cells, e.g., cells obtained from an individual, including cell lines. The present invention is not limited to samples comprising tissue sections. In some embodiments, the sample comprises nucleic acid, protein, bacteria, viruses, or any other test agent.

The samples (e.g., tissue sections) may be processed in a manner compatible with histochemical staining, including, for example, as described below, fixation, embedding in a wax matrix (such as paraffin), and sectioning (such as with a microtome). No specific processing step is required by the present disclosure, so long as the sample obtained is compatible with the methods of the present invention, e.g., histochemical staining of the sample for the biomarkers of interest.

Generally, tissue samples are prepared by fixing and embedding the tissue in a medium. In other examples, samples include a cell suspension, which is prepared as a monolayer on a solid support (such as a glass slide) for example by smearing or centrifuging cells onto the solid support. In further examples, fresh frozen (for example, unfixed) tissue sections may be used in the methods disclosed herein.

The process of fixing a sample can vary. Fixing a tissue sample preserves cells and tissue constituents in as close to a life-like state as possible and allows them to undergo preparative procedures without significant change. Fixation arrests the autolysis and bacterial decomposition processes that begin upon cell death, and stabilizes the cellular and tissue constituents so that they withstand the subsequent stages of tissue processing.

Tissues can be fixed by any suitable process, including perfusion or by submersion in a fixative. Fixatives can be classified as cross-linking agents (such as aldehydes, e.g., formaldehyde, paraformaldehyde, and glutaraldehyde, as well as non-aldehyde cross-linking agents), oxidizing agents (e.g., metallic ions and complexes, such as osmium tetroxide and chromic acid), protein-denaturing agents (e.g., acetic acid, methanol, and ethanol), fixatives of unknown mechanism (e.g., mercuric chloride, acetone, and picric acid), combination reagents (e.g., Carnoy's fixative, methacarn, Bouin's fluid, B5 fixative, Rossman's fluid, and Gendre's fluid), microwaves, and miscellaneous fixatives (e.g., excluded volume fixation and vapor fixation). Additives may also be included in the fixative, such as buffers, detergents, tannic acid, phenol, metal salts (such as zinc chloride, zinc sulfate, and lithium salts), and lanthanum.

The most commonly used fixative in preparing samples is formaldehyde, generally in the form of a formalin solution (4% formaldehyde in a buffer solution, referred to as 10% buffered formalin). In one example, the fixative is 10% neutral buffered formalin.

In some examples an embedding medium is used. An embedding medium is an inert material in which tissues and/or cells are embedded to help preserve them for future analysis. Embedding also enables tissue samples to be sliced into thin sections. Embedding media include paraffin, celloidin, OCT^{™} compound, agar, plastics, or acrylics. Many embedding media are hydrophobic; therefore, the inert material may need to be removed prior to histological or cytological analysis, which utilizes primarily hydrophilic reagents. The term deparaffinization or dewaxing is broadly used herein to refer to the partial or complete removal of any type of embedding medium from a biological sample. For example, paraffin-embedded tissue sections are dewaxed by passage through organic solvents, such as toluene, xylene, limonene, or other suitable solvents.

As an example, a tissue sample may be a FFPE tissue sample for histological examination on an automated staining machine. In this case, the sample can be deparaffinized, e.g., with an automated IHC/ISH slide stainer, using appropriate deparaffinizing fluid(s). Subsequently, any number of substances can be successively applied to the sample. The substances can be for pretreatment, cell lysis, denaturation, washing, staining, or the like.

### Antigen Retrieval

Extended fixation times can be damaging to many antigens, in addition to the induction of molecular cross-links in proteins. This can change the native protein conformation, thereby altering the normal structure of the epitope. The result is that a biomarker of interest may be masked, or in accessible to an antibody during immunohistochemistry. In some cases, the effects of fixation can be overcome using unmasking or antigen retrieval techniques. Antigen retrieval can be achieved by physical approaches, chemical approaches, or a combination of both. Examples of methods of antigen retrieval are discussed in Shi et al. (J Histochemistry & Cytochemistry, 2011, 59:13-32), D'Amico et al. (J Immunological Methods, 2009, 341:1-18), and McNicoll and Richmond (Histopathology, 1998, 32:97-103), as well we U.S. Pat. No. 9,506,928 and U.S. Pat. No. 6,544,798. For example, antigen retrieval techniques include protease digestion (e.g., trypsin, DNase, proteinase K, pepsin, pronase, ficin, etc.), which helps to cleave molecular cross-links and allow the epitope to return to its normal conformation); sonication-induced epitope retrieval (SIER); and heat induced epitope retrieval (HIER), which involves placing slides in heated solutions of various compositions before application of the primary antibody (or before other compositions are applied to the sample). Microwave ovens, pressure cookers, hot water baths, autoclaves, automated staining machines are possible means of achieving heat. HIER solutions may include 0.1M citrate buffer (pH 6), 0.1M EDTA (pH 9) (or other calcium-chelating agent solutions), 0.5M Tris base buffer (pH 10), 0.05M glycine-HCl buffer, 1% periodic acid, various concentrations of urea, lead thiocyanate solutions, etc. Various degrees of epitope retrieval can be obtained by varying heating times, heating temperatures, and pH.

### Tyramide-Chromogen Detection

Tyramide Signal Amplification (TSA) is a known method based on catalyzed reporter deposition (CARD). U.S. Patent No. 5,583,001, discloses a method for detection or quantitation of an analyte using an analyte-dependent enzyme activation system relying on catalyzed reporter deposition to amplify the reporter signal enhancing the catalysis of an enzyme in a CARD or TSA method by reacting a labeled phenol molecule with an enzyme. While tyramide signal amplification is known to amplify the visibility of targets, it is also associated with elevated background staining (e.g., amplification of non-specific recognition events).

For example, the amount of protein surrounding the target or targets may be insufficient. When detecting biomarkers present at high levels, or when detecting the co-localization of multiple biomarkers, the amount of protein in the sample to which the tyramide-based detection reagents can attach may be the limiting reagent. An insufficiency in tyramide binding sites can cause a reduced reaction rate, allow the tyramide reactive molecules to diffuse away from the target, and generally results in a weaker response due to lower quantities of the signaling conjugates reacting in the vicinity of the target.

Tyramide-chromogen conjugates have been used for miRNA detection (see U.S. Patent Application No. 2013/0260379 and WO 201 5/124738. For example, a target may be detected by a probe labeled with a hapten. An anti-hapten antibody conjugated to an enzyme then is contacted to the sample so that the antibody binds to the probe and links the enzyme to the target. The enzyme catalyzes deposition of a tyramide-hapten conjugate. A plurality of tyramide-hapten conjugate binds to the sample in the vicinity of the target, thus substantially amplifying the signal associated with target. A second enzyme-conjugated anti-hapten antibody is then contacted to the sample and allowed to bind to the tyramide-hapten conjugate. The second enzyme can then be used to catalyze deposition of a tyramide-chromogen conjugate, e.g., silver deposition (Ventana Medical Systems, Inc. Catalog #:780-001), or any other chromogen desired.

It was surprising that the tyramide amplification methods such as those for detecting mRNA could be used for histochemical detection of targets in a sample. The histochemical application requires more target (detecting antibodies instead of probes in the mRNA case), which would normally be associated with high levels of background. Previous reposts of tyramide-based signal amplification systems describe an abundance of background signal. It was surprisingly found that the tyramide-based conjugates used in the methods of the present invention did not produce too much background. Indeed, the methods of the present invention provide sensitivity (e.g., ability to accurately label the target biomarker), uniformity (e.g., ability to distribute signal uniformly on the sample so as to avoid pockets of diffuse staining), and may provide measurements of quantity (e.g., through the use of the punctate dot signal).

In certain embodiments, the concentration of tyramide conjugate in the reaction (final concentration) is from 1 to 10 µM (e.g., 3 µM). In certain embodiments, the final concentration of tyramide conjugate is from 5 to 10 µM (e.g., 7 µM). In certain embodiments, the final concentration of tyramide conjugate is from 5 to 20 µM (e.g., 7 µM). In certain embodiments, the final concentration of tyramide conjugate is from 0.1 to 1.0 µm. In certain embodiments, the final concentration of tyramide conjugate is from 10 to 25 µm. In certain embodiments, the final concentration of tyramide conjugate is more than 10 µM. In certain embodiments, the final concentration of tyramide conjugate is more than 20 µ M.

Without wishing to limit the present invention to any theory or mechanism, it was thought that polyclonal antibodies would generate off-target binding and result in background signal (because of their inherent increased ability to bind things other than the intended antigen). For example, the RNA methods require the use of a monoclonal antibody for detecting the labeled probe. It was surprisingly found that this was not observed with a polyclonal antibody used in the methods herein (the polyclonal antibody did not generate detectable background signal). Thus, in certain embodiments, a polyclonal antibody may be used for the qIHC methods herein (for detecting the target-specific binding agent, e.g., the primary antibody). In certain embodiments, a monoclonal antibody is used for the qIHC methods herein (for detecting the target-specific binding agent, e.g., the primary antibody).

### Histochemical Labeling, Biomarker-Specific Agents, and Detection Systems

Generally, biomarker (target) labeling may be accomplished by contacting a sample with a biomarker-specific reagent (e.g., antibody) under conditions that facilitate specific binding between the target biomarker and the biomarker-specific reagent. The sample is then contacted with a set of detection reagents that interact with the biomarker-specific reagent to facilitate deposition a detectable moiety in close proximity the target biomarker, thereby generating a detectable signal localized to the target biomarker. A variety of different schemes for detection are possible for typical immunohistochemistry applications.

Non-limiting examples of commercially available detection reagents or kits comprising detection reagents include: VENTANA ultraView detection systems (secondary antibodies conjugated to enzymes, including HRP and AP); Ventana iVIEW detection systems (biotinylated anti-species secondary antibodies and streptavidin-conjugated enzymes); VENTANA OptiView detection systems (OptiView) (anti-species secondary antibody conjugated to a hapten and an anti-hapten tertiary antibody conjugated to an enzyme multimer); VENTANA Amplification kit (unconjugated secondary antibodies, which can be used with any of the foregoing VENTANA detection systems to amplify the number of enzymes deposited at the site of primary antibody binding); VENTANA OptiView Amplification system (Anti-species secondary antibody conjugated to a hapten, an anti-hapten tertiary antibody conjugated to an enzyme multimer, and a tyramide conjugated to the same hapten.

Biomarker-stained sections may optionally be additionally stained with a contrast agent (such as a hematoxylin stain) to visualize macromolecular structures, identify morphological features or morphologically relevant areas, either manually or automatically. Non-limiting examples of counterstains include chromogenic nuclear counterstains, such as hematoxylin (stains from blue to violet), Methylene blue (stains blue), toluidine blue (stains nuclei deep blue and polysaccharides pink to red), nuclear fast red (also called Kernechtrot dye, stains red), and methyl green (stains green); non-nuclear chromogenic stains, such as eosin (stains pink), etc.

In the methods of the present invention, a biomarker-specific agent is applied to the sample, wherein the biomarker-specific agent is specific for and binds to the target in the sample. In some embodiments, the biomarker-specific agent is a primary antibody comprising an antibody an antibody fragment. The primary antibody may be a native, unmodified or other appropriate antibody. In some embodiments, the primary antibody is monoclonal. In some embodiments, the primary antibody is polyclonal.

Subsequently, a second binding agent is applied to the sample, wherein the second binding agent is specific for and binds to the biomarker-specific agent bound to the target. In some embodiments, the second binding agent is a secondary antibody comprising an antibody or fragment thereof. The second binding agent (e.g., secondary antibody) is conjugated to a secondary enzyme (e.g., a secondary antibody enzyme). The second binding agent (e.g., secondary antibody) is specific for a feature of the biomarker-specific agent: the second binding agent (e.g., secondary antibody) may be an anti-species antibody, an anti-modification antibody, etc., or a combination thereof. Suitable enzymes are well-known and include, but are not limited to, oxidoreductases, hydrolases, and peroxidases. Specific enzymes explicitly included are horseradish peroxidase (HRP), alkaline phosphatase (AP), acid phosphatase, glucose oxidase, β-galactosidase, β-glucuronidase, and β-lactamase.

Subsequently, a tyramide agent (e.g., a tyramide molecule conjugated with a tyramide hapten) is applied to the sample. The secondary enzyme catalyzes deposition of the tyramide hapten onto the sample. Tyramide haptens may include but are not limited to biotin, digoxigenin (DIG), NP, BF, NCA, BD, dinitrophenyl (DNP), oxazole, pyrazole, thiazole, nitroaryl, benzofuran, triperpene, urea, thiourea, rotenoid, coumarin and cyclolignan haptens, such as those disclosed in U.S. Pat. No. 7,695,929.

Subsequently, a third binding agent is applied to the sample. The third binding agent is specific for and binds to the tyramide hapten. In some embodiments, the third binding agent is a tertiary antibody comprising an antibody or a fragment thereof. The third binding agent (e.g., tertiary antibody) is conjugated with a tertiary enzyme (e.g., tertiary antibody enzyme). Suitable enzymes are well-known and include, but are not limited to, oxidoreductases, hydrolases, and peroxidases. Specific enzymes explicitly included are horseradish peroxidase (HRP), alkaline phosphatase (AP), acid phosphatase, glucose oxidase, β-galactosidase, β-glucuronidase, and β-lactamase.

Following the application of the third binding agent (e.g., tertiary antibody) specific for the tyramide hapten, a detectable moiety (e.g., chromogen) is applied to the sample. The tertiary enzyme catalyzes a reaction with the detectable moiety (e.g., chromogen) to make the detectable moiety (e.g., chromogen) visible. The detectable moiety (e.g., chromogen) may be visible as a punctate dot using microscopy, e.g., using brightfield microscopy. In certain embodiments, the punctate dotes may represent individual target molecules. Said punctate dots (e.g., target molecules) may be counted, e.g., to quantitate an amount of target molecule in the sample.

In some embodiments, the detectable moiety (e.g., chromogen) comprises silver or a tyramide-rhodamine dye. For example, in some embodiments, the tyramide-rhodamine dye comprises rhodamine 110, rhodamine 6G, tetramethyl rhodamine (TAMRA), rhodamine 6G, sulforhodamine B, sulforhodamine 101 (Texas Red), the like, or a combination thereof. The present invention is not limited to these examples of detectable moieties (e.g., chromogens). For example, without wishing to limit the present invention to any theory or mechanism, it is possible that diffuse chromogens (e.g., DAB) could work in this technology, e.g., if there are lower levels of the target molecule (biomarker) expression (the ability to resolve a single dot is not as good with diffuse chromogens when expression level is high).

As such, other non-limiting examples of detectable moieties (chromogenic compounds/substrates) include 4-nitrophenylphospate (pNPP), fast red, bromochloroindolyl phosphate (BCIP), nitro blue tetrazolium (NBT), BCIP/NBT, fast red, AP Orange, AP blue, tetramethylbenzidine (TMB), 2,2'-azino-di-[3-ethylbenzothiazoline sulphonate] (ABTS), o -dianisidine, 4-chloronaphthol (4-CN), nitrophenyl-β-D-galactopyranoside (ONPG), o-phenylenediamine (OPD), 5-bromo-4-chloro-3-indolyl-β-galactopyranoside (X-Gal), methylumbelliferyl-β-D-galactopyranoside (MU-Gal), p-nitrophenyl-o-D-galactopyranoside (PNP), 5-bromo-4-chloro-3-indolyl- β -D-glucuronide (X-Gluc), 3-amino-9-ethyl carbazol (AEC), fuchsin, iodonitrotetrazolium (INT), tetrazolium blue, or tetrazolium violet.

In some embodiments, the methods of the present invention feature a metallographic detection scheme, wherein the tertiary enzyme comprises alkaline phosphatase (or suitable alternative) and the detectable moiety (chromogen) comprises a water-soluble metal ion and a redox-inactive substrate of the tertiary enzyme. In some embodiments, the substrate is converted to a redox-active agent by the enzyme, and the redox-active agent reduces the metal ion, causing it to form a detectable precipitate. (see, for example, U.S. Patent No. 7,642,064, PCT Publication No. 2005/003777 and U.S. Patent No. 7,632,652. Metallographic detection methods include using an oxido-reductase enzyme (such as horseradish peroxidase) along with a water soluble metal ion, an oxidizing agent and a reducing agent, again to for form a detectable precipitate. (See, for example, U.S. Patent No. 6,670,1 13.

In yet other embodiments, the detectable moiety comprises a latent reactive moiety configured to react with the tertiary enzyme to form a reactive species that can bind to the sample or to other detection components. These reactive species are capable of reacting with the sample proximal to their generation, i.e. near the enzyme, but rapidly convert to a non-reactive species so that the signaling conjugate is not deposited at sites distal from the site at which the enzyme is deposited. Examples of latent reactive moieties include: quinone methide (QM) analogs, such as those described at WO2015124703A1, and tyramide conjugates, such as those described at, WO2012/003476A2. In some examples, the latent reactive moiety is directly conjugated to a dye, such as N,N'-biscarboxypentyl-5,5'-disulfonato-indo-dicarbocyanine (Cy5), 4-(dimethylamino) azobenzene-4'-sulfonamide (DABSYL), tetramethylrhodamine (DISCO Purple), and Rhodamine 110 (Rhodamine).

Note in some embodiments, the methods of the present invention are applied in a multiplex staining method for detecting multiple target biomarkers. In multiplex methods, the biomarker-specific reagents and detection reagents are applied in a manner that allows the different biomarkers to be differentially labeled.

One way to accomplish differential labeling of different biomarkers is to select combinations of biomarker-specific reagents, detection reagents, and enzyme combinations that will not result in off-target cross-reactivity between different antibodies or detection reagents (termed "combination staining"). For example, where secondary detection reagents are used, each secondary detection reagent is capable of binding to only one of the primary antibodies used on the section. For example, primary antibodies could be selected that are derived from different animal species (such as mouse, rabbit, rat, and got antibodies), in which case species-specific secondary antibodies may be used. As another example, each primary antibody may include a different hapten or epitope tag, and the secondary antibodies are selected to specifically bind to the hapten or epitope tag. Additionally, each set of detection reagents should be adapted to deposit a different detectable entity on the section, such as by depositing a different enzyme in proximity to each biomarker-specific reagent. An example of such an arrangement is shown at US 8,603,765. Such arrangements have the potential advantage of being able to have each set of biomarker-specific reagents and associated specific binding reagents present on the sample at the same time and/or to perform staining with cocktails of biomarker-specific reagents and detection reagents, thereby reducing the number of staining steps. However, such arrangements may not always be feasible, as reagents may cross-react with different enzymes, and the various antibodies may cross-react with one another, leading to aberrant staining.

Another way to accomplish differential labeling of different biomarkers is to sequentially stain the sample for each biomarker. In such an embodiment, a first biomarker-specific reagent is reacted with the section, followed by a secondary detection reagent to the first biomarker-specific reagent and other detection reagents resulting in deposition of a first detectable entity. The section is then treated to remove the biomarker-specific reagents and associated detection reagents from the section while leaving the deposited stain in place. The process is repeated for subsequent biomarker-specific reagent. Examples of methods for removing the biomarker-specific reagents and associated detection reagents include heating the sample in the presence of a buffer that elutes the antibodies from the sample (termed a "heat-kill method"), such as those disclosed by Stack et al., Multiplexed immunohistochemistry, imaging, and quantitation: A review, with an assessment of Tyramide signal amplification, multispectral imaging and multiplex analysis, Methods, Vol. 70, Issue 1, pp 46-58 (Nov. 2014), and PCT/EP2016/057955.

As will be appreciated by the skilled artisan, combination staining and sequential staining methods may be combined. For example, where only a subset of the primary antibodies are compatible with combination staining, the sequential staining method can be modified, wherein the antibodies compatible with combination staining are applied to the sample using a combination staining method, and the remaining antibodies are applied using a sequential staining method.

In some embodiments, the methods of the present invention are used to detect two target molecules in the same sample. In some embodiments, the methods of the present invention are used to detect three target molecules in the same sample. In some embodiments, the methods of the present invention are used to detect four target molecules in the same sample. In some embodiments, the methods of the present invention are used to detect five target molecules in the same sample. In some embodiments, the methods of the present invention are used to detect six target molecules in the same sample. In some embodiments, the methods of the present invention are used to detect seven target molecules in the same sample. In some embodiments, the methods of the present invention are used to detect eight or more target molecules in the same sample.

### Automated Systems

The methods of the present invention may be performed on an automated staining machine (slide stainer) or other appropriate automated slide processing machine. Specific examples of automated staining machines (e.g., IHC/ISH slide stainers) include: itelliPATH (Biocare Medical), WAVE (Celerus Diagnostics), DAKO OMNIS and DAKO AUTOSTAINER LINK 48 (Agilent Technologies), BENCHMARK (Ventana Medical Systems, Inc.), Leica BOND, and Lab Vision Autostainer (Thermo Scientific). Automated staining machines (automated slide stainers) are also described by Prichard, Overview of Automated Immunohistochemistry, Arch Pathol Lab Med., Vol. 138, pp. 1578-1582 (2014). Additionally, Ventana Medical Systems, Inc. is the assignee of a number of United States patents disclosing systems and methods for performing automated analyses, including U.S. Pat. Nos. 5,650,327, 5,654,200, 6,296,809, 6,352,861, 6,827,901 and 6,943,029, and U.S. Published Patent Application Nos. 20030211630 and 20040052685. The methods of the present invention may be adapted to be performed on any appropriate automated staining machine (or automated slide processing machine).

Automated IHC/ISH slide stainers typically include at least a stainer unit for dispensing reagent to implement staining protocols onto a slide. Commercially-available staining units typically operate on one of the following principles: (1) open individual slide staining, in which slides are positioned horizontally and reagents are dispensed as a puddle on the surface of the slide containing a tissue sample (such as implemented on the DAKO AUTOSTAINER Link 48 (Agilent Technologies) and intelliPATH (Biocare Medical) stainers); (2) liquid overlay technology, in which reagents are either covered with or dispensed through an inert fluid layer deposited over the sample (such as implemented on BenchMark and VENTANA DISCOVERY stainers); (3) capillary gap staining, in which the slide surface is placed in proximity parallel to another surface (which may be another slide or a coverplate) to create a narrow gap, through which capillary forces draw up and keep liquid reagents in contact with the samples (such as the staining principles used by DAKO TECHMATE, Leica BOND, and DAKO OMNIS stainers). Some iterations of capillary gap staining do not mix the fluids in the gap (such as on the DAKO TECHMATE and the Leica BOND). In some variations of capillary gap staining, the reagents are mixed in the gap, such as translating gap technology, in which a gap is created between the slide and a curved surface and movement of the surfaces relative to one another effects mixing (see US 7,820,381); and dynamic gap staining, which uses capillary forces similar to capillary gap staining to apply sample to the slide, and then translates the parallel surfaces relative to one another to agitate the reagents during incubation to effect reagent mixing (such as the staining principles implemented on DAKO OMNIS slide stainers (Agilent)). It has recently been proposed to use inkjet technology to deposit reagents on slides. See WO 2016-170008 A1. This list of staining principles is not intended to be exhaustive, and the present methods and systems are intended to include any staining technology (both known and to be developed in the future) that can be used to apply the appropriate reagents to the sample.

The present invention is not limited to methods applied in automated systems. In some embodiments, the methods of the present invention are applied manually.

Without wishing to limit the present invention to any theory or mechanism, it is believed that the methods of the present invention, which provide for quantification of the signals and expression levels of target molecules, may help provide additional prognostic value and/or predictive value for patients as compared to the traditional methods that generate a binary (plus or minus) result. Further, the methods of the present invention may provide for standardization of IHC assays. The methods of the present invention may be used for multiple target molecules.

### Image Processing and Analysis

Following staining of the tissue section, samples undergo image acquisition, image processing, and analysis.

Digital image acquisition systems may comprise a scanning platform such as a slide scanner that can scan the stained slides at 20x, 40x, or other magnifications to produce high resolution whole-slide digital images, including for example slide scanners. At a basic level, the typical slide scanner includes at least: (1) a microscope with lens objectives, (2) a light source (such as halogen, light emitting diode, white light, and/or multispectral light sources, depending on the dye), (3) robotics to move glass slides around (or to move the optics around the slide), (4) one or more digital cameras for image capture, (5) a computer and associated software to control the robotics and to manipulate, manage, and view digital slides. Digital data at a number of different X-Y locations (and in some cases, at multiple Z planes) on the slide are captured by the camera's charge-coupled device (CCD), and the images are joined together to form a composite image of the entire scanned surface. Common methods to accomplish this include: (1) Tile based scanning, in which the slide stage or the optics are moved in very small increments to capture square image frames, which overlap adjacent squares to a slight degree. The captured squares are then automatically matched to one another to build the composite image; and (2) Line-based scanning, in which the slide stage moves in a single axis during acquisition to capture a number of composite image "strips." The image strips can then be matched with one another to form the larger composite image.

In some embodiments, the imaging apparatus is a brightfield imager slide scanner. One brightfield imager is the iScan Coreo brightfield scanner sold by Ventana Medical Systems, Inc. In automated embodiments, the imaging apparatus is a digital pathology device as disclosed in International Patent Application No.: PCT/US2010/002772 (Patent Publication No.: WO/2011/049608) entitled IMAGING SYSTEM AND TECHNIQUES or disclosed in U.S. Patent Application No. 61/533,114, filed on Sep. 9, 2011, entitled IMAGING SYSTEMS, CASSETTES, AND METHODS OF USING THE SAME. International Patent Application No. PCT/US2010/002772 and U.S. Patent Application No. 61/533,1 14.

A detailed overview of various scanners (e.g., brightfield) can be found at Farahani et al., Whole slide imaging in pathology: advantages, limitations, and emerging perspectives, Pathology and Laboratory Medicine Int'l, Vol. 7, p. 23-33 (June 2015), the content of which is incorporated by reference in its entirety. Examples of commercially available slide scanners include: 3DHistech PANNORAMIC SCAN II; DigiPath PATHSCOPE; Hamamatsu NANOZOOMER RS, HT, and XR; Huron TISSUESCOPE 4000, 4000XT, and HS; Leica SCANSCOPE AT, AT2, CS, FL, and SCN400; Mikroscan D2; Olympus VS120-SL; Omnyx VL4, and VL120; PerkinElmer LAMINA; Philips ULTRA-FAST SCANNER; Sakura Finetek VISIONTEK; Unic PRECICE 500, and PRECICE 600x; VENTANA ISCAN COREO and ISCAN HT; and Zeiss AXIO SCAN.Z1. Other exemplary systems and features can be found in, for example, WO2011-049608) or in U.S. Patent Application No. 61/533,114, filed on Sep. 9, 2011, entitled IMAGING SYSTEMS, CASSETTES, AND METHODS OF USING THE SAME.

Images generated by the scanning platform may be transferred to an image analysis system or to a server or database accessible by an image analysis system. In some embodiments, the images may be transferred automatically via one or more local-area networks and/or wide-area networks. In some embodiments, the image analysis system may be integrated with or included in the scanning platform and/or other modules of the image acquisition system, in which case the image may be transferred to the image analysis system. In some embodiments, the image acquisition system may not be communicatively coupled to the image analysis system, in which case the images may be stored on a non-volatile storage medium of any type (e.g., a flash drive) and downloaded from the medium to the image analysis system or to a server or database communicatively coupled thereto.

The image acquisition system may also be integrated into an automated slide staining machine or automated slide processing system and/or an automated H&E staining platform (as described above).

In a simplex staining method, IHC is performed on individual serial sections, which are then individually imaged. Once imaged, feature extraction is performed in order to align the sections for subsequent analysis. Software for aligning the tissue sections is well known to one of ordinary skill in the art.

In a multiplex staining method, IHC and image acquisition is performed on a single section. Because the tissue section features multiple biomarkers (e.g., multiple chromogens, etc.), the image is processed to separate out the different signals. For example, processing of the multiplexed stained tissue section image may feature unmixing (also known as spectral unmixing, color separation, color deconvolution, etc.) of the digital image into its individual constituent dyes for each biomarker and obtaining the proportion of each dye in the color mixture. For example, the process may comprise unmixing the digital image into a first deconstructed image for a first chromogen (first biomarker), a second deconstructed image for a second chromogen (second biomarker), etc. Typically, the image acquisition obtains a RGB color image, which is a mixture of the underlying co-localized biomarker expressions. Several techniques have been proposed to decompose each pixel of the RGB image into a collection of constituent stains and the fractions of the contributions from each of them. Ruifrok et al., (Anal. Quant. Cytol. Histol., 2001, 23:291-299) developed an unmixing method called color deconvolution to unmix the RGM image with up to three stains. Other methods for unmixing of multi-spectral images includes those disclosed in Chen and Srinivas (Comput Med Imaging Graph, 2015, 46(1):30-39), Kesheva (Lincoln Laboratory Journal, 2003, 14:55-78), Greer (IEEE Trans Image Proc., 2012, 221:219-228), and Yang et al. (IEEE Trans. Image Proc., 2011, 20:1112-1125).

The unmixing process extracts stain-specific channels to determine local concentrations of individual stains using color reference vectors, or reference spectra, that are well-known for standard types of tissue and stain combinations. Each pixel of a scanned image is represented by a vector of image values, or a color vector, and each stain corresponds to a color reference vector. The local concentration of the stain is represented by a scaling factor of a color reference vector. Therefore, the color vector for a pixel that contains multiple co-located stains with different concentrations is a linear combination of the reference spectra of all the present stains. In brightfield (transmission) imaging, light intensities emitted by the stained tissue are transformed into an optical density space, with mixing of different stains being represented by a linear weighted combination of the contributing reference spectra.

In certain embodiments, the stained tissue sections may comprise a concentration-dependent stain (e.g., a stain that has different chromatic properties at different concentrations). Thus, the methods of unmixing the digital image of the tissue section may account for the effects of light scattering and how, at varying stain concentrations, light scattering may change the proportions of RGB channel signals in detected light. This may feature selecting an optimal color reference vector for the concentration-dependent stain selected from a set of color reference vectors for the concentration-dependent stain (wherein each color reference vector in the set describes or characterizes the concentration-dependent stain at a different concentration level), and unmixing the image using the selected optimal color reference vector.

In certain embodiments, a region of interest (ROI) is manually identified in the digital image. For example, a trained expert may manually delineate one or more morphological region(s) on a digital image of the sample. In other embodiments, a computer-implemented system may assist the user in annotating the ROI (termed, "semi-automated ROI annotation"). For example, the user may delineate one or more regions on the digital image, which the system then automatically transforms into a complete ROI. For example, if the desired ROI is an invasive margin (IM) region, a user can delineate (e.g., by outlining, tracing) an IM region or a whole tumor (WT) region, and the system applies a pattern recognition function that uses computer vision and machine learning to identify regions having similar morphological characteristics to an IM region. Many other arrangements could be used as well. In cases in which ROI generation is semi-automated, the user may be given an option to modify the ROI annotated by the computer system, such as by expanding the ROI, annotating regions of the ROI or objects within the ROI to be excluded from analysis, etc. In other embodiments, the computer system may automatically suggest an ROI without any direct input from the user (termed an "automated ROI annotation"). For example, a previously trained tissue segmentation function or other pattern recognition function may be applied to an unannotated image to identify the desired morphological region to use as an ROI. The user may be given an option to modify the ROI annotated by the computer system, such as by expanding the ROI, annotating regions of the ROI or objects within the ROI to be excluded from analysis, etc. For a simplex serial section of an H&E slide, a tumor region may be identified (annotated) and the annotation transferred to the other serial images in a process called "registering" of the image. Registration may also be done with a multiplex assay.

Image analysis systems may feature one or more computing devices such as desktop computers, laptop computers, tablets, smartphones, servers, application-specific computing devices, or any other type(s) of electronic device(s) capable of performing the techniques and operations described herein. In some embodiments, the image analysis system may be implemented as a single device. In other embodiments, the image analysis system may be implemented as a combination of two or more devices together. For example, an image analysis system may include one or more server computers and a one or more client computers communicatively coupled to each other via one or more local-area networks and/or wide-area networks such as the Internet.

The image analysis system may include a memory, a processor, and a display. The memory may include any combination of any type of volatile or non-volatile memories, such as random-access memories (RAMs), read-only memories such as an Electrically-Erasable Programmable Read-Only Memory (EEPROM), flash memories, hard drives, solid state drives, optical discs, and the like. The processor may include one or more processors of any type, such as central processing units (CPUs), graphics processing units (GPUs), special-purpose signal or image processors, field-programmable gate arrays (FPGAs), tensor processing units (TPUs), and so forth.

The display may be implemented using any suitable technology, such as LCD, LED, OLED, TFT, Plasma, etc. In some implementations, display may be a touch-sensitive display (a touchscreen).

The image analysis system may also include an object identifier, a region of interest (ROI) generator, a user-interface module, and a scoring engine. It can be appreciated by persons having ordinary skill in the art that each module may be implemented as a number of sub-modules, and that any two or more modules can be combined into a single module. Furthermore, in some embodiments, the system may include additional engines and modules (e.g., input devices, networking and communication modules, etc.). Exemplary commercially-available software packages useful in implementing modules as disclosed herein include VENTANA VIRTUOSO; Definiens TISSUE STUDIO, DEVELOPER XD, and IMAGE MINER; and Visopharm BIOTOPIX, ONCOTOPIX, and STEREOTOPIX software packages.

After acquiring an image, the image analysis system may pass the image to an object identifier, which functions to identify and mark relevant objects and other features within the image that will later be used for scoring. In the present invention, biomarkers appear as punctate dots that can be quantified. Thus, the objects identified in the image analysis are the punctate dots within the ROI (e.g., whole tumor, invasive margin, tumor core, peri-tumoral region, etc.).

The object identifier and the annotation of the ROI (ROI generator) may be implemented in any order. For example, the object identifier may be applied to the entire image first. The positions and features of the identified objects may then be stored and recalled when the ROI generator is implemented. A score can be generated by a scoring engine upon generation of the ROI. Alternatively, the ROI generator can be implemented first. In this case, the object identifier may be implemented only on the ROI, or it may still be implemented on the whole image. It may also be possible to implement the object identifier and the ROI generator simultaneously.

After both the object identifier and the ROI generator are implemented, a scoring engine is implemented.

FIG. 6 shows punctate dot signals observed at low to medium expression levels of HER2 (bottom pane) using the methods of the present invention (qIHC) compared to the diffuse staining using traditional methods (top panel). The punctate dots are distinct from diffuse staining, since diffuse staining covers a larger area of the tissue sample. The methods herein provide scoring of a tissue section using the punctate dot signals for one or more biomarkers labeled in the tissue section. For example, image analysis software may input the quantitated biomarkers (object metric) into a pre-determined scoring function to obtain a score for the tissue section.

The present invention also provides computing systems and computer algorithms for use with automated systems described herein.

### EXAMPLE 1

Example 1 describes a series of experiments using methods of the present invention. The present invention is not limited to the methods, systems, and compositions described herein.

Experiment 1: A high-sensitivity detection system (a method of the present invention) was assembled including the V5-epitope tagged 4B5 anti-Her2 rabbit monoclonal antibody + a mouse anti-V5-HRP conjugate + tyramide-DIG + mouse anti-DIG-HRP conjugate + silver chromogen. Determination of the anti-V5-HRP conjugate concentration that enables specific (little or no background) and sensitive detection of anti-Her2 antibody bound to Her2-expressing cells in each tissue sample was accomplished using with a titration experiment. Tonsil and Her2 3in1 xenograft slides were stained using V5-epitope tagged 4B5 anti-Her2 rabbit monoclonal antibody or a negative control (antibody diluent) and a range of anti-V5-HRP conjugate concentrations. A similar experiment was executed to demonstrate specificity (little or no background signal) for the tyramide-DIG + mouse anti-DIG-HRP conjugate + silver chromogen reagents (omitted the mouse anti-V5-HRP conjugate).

Experiment 2: Repeated Experiment 1 using optimal reagent concentrations and five tonsil and five Her2 3in1 xenograft slides. Results demonstrated increased sensitivity of the high-sensitivity detection system (method of the present invention) compared to the RTD OptiView amp DAB detection system.

Experiment 3: Experiment executed to evaluate utility to replace the silver chromogen with tyramide-rhodamine dyes to generate punctate dot signals. The high-sensitivity detection system (method of the present invention) was utilized, including the V5-epitope tagged 4B5 anti-Her2 rabbit monoclonal antibody + a mouse anti-V5-HRP conjugate + tyramide-DIG + mouse anti-DIG-HRP conjugate + tyramide-rhodamine dye chromogens, to stain tonsil and Her2 3in1 xenograft slides. No primary antibody slides served as negative control slides. Punctate dot signals were observed for three different tyramide-rhodamine dye chromogens (tyramide-Rhodamine 6G, tyramide-tetramethylrhodamine (TAMRA), and tyramide-sulforhodamine 101 (Texas Red). Utility to generate punctate dot signals suggests an ability to adapt the high-sensitivity protein detection system to a multiplex system using chromogen dyes with narrow absorbance spectra unmixible using computer algorithms.

Experiment 4: Experiment executed to evaluate utility of the high-sensitivity protein detection system (method of the present invention) for detection of secreted protein factors. Interferon gamma (IFNg) was selected as a model secreted factor due to its well-documented biological role in leukocyte (T cell) biology and the inflammatory process (likely expressed by T and NKT cells in reactive tonsil tissues). A polyclonal native (un-modified) rabbit anti-human IFNg was purchased (Abcam) and used to stain human three human tonsil tissues (presumed positive samples) and Her2 3in1 xenograft tissue (presumed negative control tissues as the xenograft tissues are comprised of human cancer cell lines grown in immuno-deficient, SCID, mice which lack T and NKT cells. Titration experiments had been previously executed to determine appropriate concentration of the RTD OmniMap polyclonal Goat-anti-Rabbit-HRP conjugate for sensitive and specific detection of rabbit antibodies using the high-sensitivity detection system (method of the present invention) (tyramide-DIG + mouse anti-DIG-HRP conjugate + silver chromogen); 0.25x of the commercially sold reagent generated little to no background signal). Utility of the high-sensitivity system for detection of IFNg secreted from immune cells was observed as intense signal surrounding few cells and more diffuse signals emanating from other cells; specificity of the technology for detection of secreted factors was supported by lack of signals in a majority of tonsil cells and lack of signal on the Her2 3in1 xenograft tissues. FIG. 7 shows the results of immunohistochemistry of IFNg in reactive tonsil using traditional methods of IHC (top panels) and methods of the present invention, qIHC (bottom panels). Note the punctate dot signals of IFN gamma observed using the methods of the present invention (qIHC) compared to the diffuse staining using traditional methods. This suggests the methods of the present invention may be used for secreted target molecules.

Experiment 5: Experiment executed test hypothesis that the high-sensitivity protein detection technology (method of the present invention) enables detection of individual antibodies bound to single protein molecules. A two-color detection system was assembled to include the following reagents: V5-epitope tagged 4B5 anti-Her2 rabbit monoclonal antibody + a mouse anti-V5-HRP conjugate + tyramide-DIG + mouse anti-DIG-HRP conjugate + tyramide-TAMRA chromogen (Red signal) AND E2-epitope tagged 4B5 anti-Her2 rabbit monoclonal antibody + a rabbit anti-E2-HRP conjugate + tyramide-NP + mouse anti-NP-HRP conjugate + silver chromogen (black signal). Experiments conducted previously had determined appropriate concentrations of E2-HRP and NP-based detection reagents for generation of sensitive and specific signals using models similar to those used to optimize V5 and DIG detection reagents configurations. Utility of the high-sensitivity detection technology (method of the present invention) to generate signals from individual antibodies bound to single protein molecules was supported by the generation of individual black or red signals in Her2-expressing cells in either tonsil or Her2 3in1 xenograft tissues (ZR75.1 xenograft).

### EXAMPLE 2

Referring to FIG. 9, a multiplex staining assay was used to stain tonsil tissue for Her2. A V5 epitope-tagged anti-Her2 monoclonal antibody 4B5 was detected using Mouse anti-V5-HRP + tyramide-DIG + Mouse anti-DIG-HRP + tyramide-TAMRA chromogen (pink). An E2 epitope-tagged anti-Her2 monoclonal antibody 4B5 was detected using Mouse anti-E2-HRP + tyramide-NP + Mouse anti-NP-HRP + Silver chromogen (black). Note, the V5 and E2 tagged 4B5 anti-Her2 monoclonal antibodies compete for the same amino acid sequence in the target Her2 protein and were co-incubated together before detection was executed. The single pink punctate dot signals likely derived from individual single V5-labeled 4B5 mAb bound to target; each antibody likely binds only a single Her2 protein. FIG. 9 is consistent with detection of individual antibodies bound to an individual epitope (e.g., single protein molecule detection). FIG. 10 shows detection of Her2 protein in tonsil tissue demonstrating specificity of the assay. The left panel shows the control sample and the right panel shows the sample stained for Her2. The detection system used was the OmniMap xRBT-HRP(0.25x), tyr-DIG, xDIG-HRP, and silver (Ag).

Various modifications of the invention, in addition to those described herein, will be apparent to those skilled in the art from the foregoing description. Such modifications are also intended to fall within the scope of the appended claims.

Although there has been shown and described the preferred embodiment of the present invention, it will be readily apparent to those skilled in the art that modifications may be made thereto which do not exceed the scope of the appended claims. Therefore, the scope of the invention is only to be limited by the following claims. Reference numbers recited in the claims are exemplary and for ease of review by the patent office only, and are not limiting in any way. In some embodiments, the figures presented in this patent application are drawn to scale, including the angles, ratios of dimensions, etc. In some embodiments, the figures are representative only and the claims are not limited by the dimensions of the figures. In some embodiments, descriptions of the inventions described herein using the phrase "comprising" includes embodiments that could be described as "consisting of", and as such the written description requirement for claiming one or more embodiments of the present invention using the phrase "consisting of" is met.

## Claims

1. A method for amplifying a signal for a target molecule in a sample, the method comprising:
a. applying to the sample a first binding agent specific for the target molecule;
b. applying to the sample a second binding agent specific for the first binding agent, wherein the second binding agent is conjugated with a secondary enzyme;
c. applying to the sample a tyramide agent comprising a tyramide molecule conjugated with a hapten, wherein the secondary enzyme catalyzes deposition of the hapten onto the sample at the location of the second binding agent;
d. applying to the sample a third binding agent specific for the hapten, wherein the third binding agent is conjugated with a tertiary enzyme; and
e. applying to the sample a detectable moiety, wherein the tertiary enzyme catalyzes a reaction with the detectable moiety to make the detectable moiety visible,
wherein the detectable moiety comprises a tyramide-rhodamine dye.

2. The method according to claim 1, wherein the detectable moiety is visible as a punctate dot using brightfield microscopy, wherein the punctate dot is indicative of the target molecule.

3. The method according to claim 2, wherein the tyramide-rhodamine dye comprises rhodamine 110, rhodamine 6G, tetramethyl rhodamine (TAMRA), rhodamine 6G, sulforhodamine B, sulforhodamine 101 (Texas Red), or a combination thereof.

4. The method according to any one of claims 1 to 3, wherein the detectable moieties includes a DAB, 4-nitrophenylphospate (pNPP), fast red, bromochloroindolyl phosphate (BCIP), nitro blue tetrazolium (NBT), BCIP/NBT, fast red, AP Orange, AP blue, tetramethylbenzidine (TMB), 2,2'-azino-di-[3-ethylbenzothiazoline sulphonate] (ABTS), o -dianisidine, 4-chloronaphthol (4-CN), nitrophenyl-β-D-galactopyranoside (ONPG), o-phenylenediamine (OPD), 5-bromo-4-chloro-3-indolyl-β-galactopyranoside (X-Gal), methylumbelliferyl-β-D-galactopyranoside (MU-Gal), p-nitrophenyl-α-D-galactopyranoside (PNP), 5-bromo-4-chloro-3-indolyl- β -D-glucuronide (X-Gluc), 3-amino-9-ethyl carbazol (AEC), fuchsin, iodonitrotetrazolium (INT), tetrazolium blue, or tetrazolium violet.

5. The method according to any one of claims 1 to 4, wherein the sample is a tissue sample.

6. The method according to claim 5, wherein the tissue sample is a formalin-fixed paraffin-embedded (FFPE) tissue sample.

7. The method according to any one of claims 1 to 6, wherein the first binding agent is a primary antibody, preferably wherein the primary antibody is a monoclonal antibody.

8. The method according to any one of claims 1 to 7, wherein the second binding agent is a secondary antibody.

9. The method according to any one of claims 1 to 8, wherein the secondary enzyme is an oxidoreductase, hydrolase, or peroxidase, optionally wherein the peroxidase is horseradish peroxidase (HRP).

10. The method according to any one of claims 1 to 9, wherein the hapten is biotin, digoxigenin (DIG), NP, BF, NCA, BD, dinitrophenyl (DNP), oxazole, pyrazole, thiazole, nitroaryl, benzofuran, triperpene, urea, thiourea, rotenoid, coumarin or a cyclolignan hapten.

11. The method according to any one of claims 1 to 10, wherein the third binding agent is a tertiary antibody.

12. The method according to any one of claims 1 to 11, wherein the tertiary enzyme is alkaline phosphatase (AP).

13. The method according to any one of claims 1 to 12, wherein the method is applied in a multiplex staining method for detecting multiple target biomarkers.

14. The method according to any one of claims 1 to 13, wherein the method is performed on an automated staining machine.

15. An automated staining machine comprising a system adapted to perform a method of any one of claims 1 to 14, wherein the automated staining machine comprises a memory coupled to a processor, wherein the memory stores computer-readable instructions that, when executed by the processor, cause the automated staining machine to perform operations for the method according to any one of claims 1 to 14.

## Patentansprüche

1. Verfahren zum Amplifizieren eines Signals für ein Zielmolekül in einer Probe, wobei das Verfahren Folgendes umfasst:
a. Aufbringen eines für das Zielmolekül spezifischen ersten Bindemittels auf die Probe,
b. Aufbringen eines für das erste Bindemittel spezifischen zweiten Bindemittels auf die Probe, wobei das zweite Bindemittel mit einem sekundären Enzym konjugiert ist;
c. Aufbringen eines Tyramidmittels, umfassend ein mit einem Hapten konjugiertes Tyramidmolekül, auf die Probe, wobei das sekundäre Enzym die Abscheidung des Haptens auf die Probe an der Stelle des zweiten Bindemittels katalysiert,
d. Aufbringen eines für das Hapten spezifischen dritten Bindemittels auf die Probe, wobei das dritte Bindemittel mit einem tertiären Enzym konjugiert ist; und
e. Aufbringen einer nachweisbaren Einheit auf die Probe, wobei das tertiäre Enzym eine Reaktion mit der nachweisbaren Einheit katalysiert, um die nachweisbare Einheit sichtbar zu machen,
wobei die nachweisbare Einheit einen Tyramid-Rhodamin-Farbstoff umfasst.

2. Verfahren nach Anspruch 1, wobei die nachweisbare Einheit unter Verwendung von Hellfeldmikroskopie als ein getüpfelter Punkt sichtbar ist, wobei der getüpfelte Punkt das Zielmolekül anzeigt.

3. Verfahren nach Anspruch 2, wobei der Tyramid-Rhodamin-Farbstoff Rhodamin 110, Rhodamin 6G, Tetramethylrhodamin (TAMRA), Rhodamin 6G, Sulforhodamin B, Sulforhodamin 101 (Texas Red) oder eine Kombination davon umfasst.

4. Verfahren nach einem der Ansprüche 1 bis 3, wobei die nachweisbare Einheit ein DAB, 4-Nitrophenylphospat (pNPP), Echtrot, Bromchlorindolylphosphat (BCIP), Nitro-Blau-Tetrazolium (NBT), BCIP/NBT, Echtrot, AP-Orange, AP-Blau, Tetramethylbenzidin (TMB), 2,2'-Azino-di-[3-ethylbenzothiazolinsulfonat] (ABTS), o-Dianisidin, 4-Chlornaphthol (4-CN), Nitrophenyl-β-D-galactopyranosid (ONPG), o-Phenylendiamin (OPD), 5-Brom-4-chlor-3-indolyl-b-galactopyranosid (X-Gal), Methylumbelliferyl-b-D-galactopyranosid (MU-Gal), p-Nitrophenyl-a-D-galactopyranosid (PNP), 5-Brom-4-chlor-3-indolyl-b-D-glucuronid (X-Gluc), 3-Amino-9-ethylcarbazol (AEC), Fuchsin, Iodnitrotetrazolium (INT), Tetrazolium-Blau oder Tetrazolium-Violett einschließt.

5. Verfahren nach einem der Ansprüche 1 bis 4, wobei die Probe eine Gewebeprobe ist.

6. Verfahren nach Anspruch 5, wobei die Gewebeprobe eine formalinfixierte paraffineingebettete (FFPE) Gewebeprobe ist.

7. Verfahren nach einem der Ansprüche 1 bis 6, wobei das erste Bindemittel ein primärer Antikörper ist, vorzugsweise wobei der primäre Antikörper ein monoklonaler Antikörper ist.

8. Verfahren nach einem der Ansprüche 1 bis 7, wobei das zweite Bindemittel ein sekundärer Antikörper ist.

9. Verfahren nach einem der Ansprüche 1 bis 8, wobei das sekundäre Enzym eine Oxidoreductase, Hydrolase oder Peroxidase ist, gegebenenfalls wobei die Peroxidase Meerrettichperoxidase (HRP) ist.

10. Verfahren nach einem der Ansprüche 1 bis 9, wobei das Hapten Biotin, Digoxigenin (DIG), NP, BF, NCA, BD, Dinitrophenyl (DNP), Oxazol, Pyrazol, Thiazol, Nitroaryl, Benzofuran, Triperpen, Harnstoff, Thioharnstoff, Rotenoid, Coumarin oder ein Cyclolignanhapten ist.

11. Verfahren nach einem der Ansprüche 1 bis 10, wobei das dritte Bindemittel ein tertiärer Antikörper ist.

12. Verfahren nach einem der Ansprüche 1 bis 11, wobei das tertiäre Enzym alkalische Phosphatase (AP) ist.

13. Verfahren nach einem der Ansprüche 1 bis 12, wobei das Verfahren in einem Multiplex-Färbeverfahren zum Nachweisen mehrerer Zielbiomarker angewandt wird.

14. Verfahren nach einem der Ansprüche 1 bis 13, wobei das Verfahren auf einem Färbeautomaten durchgeführt wird.

15. Färbeautomat, umfassend ein System, das zum Durchführen eines Verfahrens nach einem der Ansprüche 1 bis 14 ausgelegt ist, wobei der Färbeautomat einen an einen Prozessor gekoppelten Speicher umfasst, wobei der Speicher computerlesbare Anweisungen speichert, die bei der Ausführung von dem Prozessor den Färbeautomaten veranlassen, Operationen für das Verfahren nach einem der Ansprüche 1 bis 14 durchzuführen.

## Revendications

1. Procédé d'amplification d'un signal pour une molécule cible dans un échantillon, le procédé comprenant :
a. l'application à l'échantillon d'un premier agent de liaison spécifique pour la molécule cible ;
b. l'application à l'échantillon d'un deuxième agent de liaison spécifique pour le premier agent de liaison, dans lequel le deuxième agent de liaison est conjugué à une enzyme secondaire ;
c. l'application à l'échantillon d'un agent tyramide comprenant une molécule tyramide conjuguée à un haptène, dans lequel l'enzyme secondaire catalyse le dépôt de l'haptène sur l'échantillon à l'emplacement du deuxième agent de liaison ;
d. l'application à l'échantillon d'un troisième agent de liaison spécifique pour l'haptène, dans lequel le troisième agent de liaison est conjugué à une enzyme tertiaire ; et
e. l'application à l'échantillon d'une fraction détectable, l'enzyme tertiaire catalysant une réaction avec la fraction détectable pour rendre la fraction détectable visible,
dans lequel la fraction détectable comprend un colorant tyramide-rhodamine.

2. Procédé selon la revendication 1, dans lequel la fraction détectable est visible en tant que point ponctuel en utilisant une microscopie à fond clair, dans lequel le point ponctuel indique la molécule cible.

3. Procédé selon la revendication 2, dans lequel le colorant tyramide-rhodamine comprend la rhodamine 110, la rhodamine 6G, la tétraméthylrhodamine (TAMRA), la rhodamine 6G, la sulforhodamine B, la sulforhodamine 101 (Texas Red), ou une combinaison de celles-ci.

4. Procédé selon l'une quelconque des revendications 1 à 3, dans lequel la fraction détectable comprend une DAB, le phosphate de 4-nitrophényle (pNPP), le fast red, le phosphate de bromochloroindolyle (BCIP), le bleu nitré-tétrazolium (NBT), le BCIP/NBT, le fast red, l'AP orange, l'AP bleu, la tétraméthylbenzidine (TMB), le 2,2'-azino-di-[sulfonate de 3-éthylbenzothiazoline] (ABTS), l'o-dianisidine, le 4-chloronaphtol (4-CN), le nitrophényl-β-D-galactopyranoside (ONPG), l'o-phénylènediamine (OPD), le 5-bromo-4-chloro-3-indolyl-β-galactopyranoside (X-Gal), le méthylumbelliféryl-β-D-galactopyranoside (MU-Gal), le p-nitrophényl-α-D-galactopyranoside (PNP), le 5-bromo-4-chloro-3-indolyl-β-D-glucuronide (X-Gluc), le 3-amino-9-éthyl-carbazol (AEC), la fuchsine, l'iodonitrotétrazolium (INT), le bleu de tétrazolium ou le violet de tétrazolium.

5. Procédé selon l'une quelconque des revendications 1 à 4, dans lequel l'échantillon est un échantillon de tissu.

6. Procédé selon la revendication 5, dans lequel l'échantillon de tissu est un échantillon de tissu fixé au formol et inclus en paraffine (FFPE).

7. Procédé selon l'une quelconque des revendications 1 à 6, dans lequel le premier agent de liaison est un anticorps primaire, de préférence dans lequel l'anticorps primaire est un anticorps monoclonal.

8. Procédé selon l'une quelconque des revendications 1 à 7, dans lequel le deuxième agent de liaison est un anticorps secondaire.

9. Procédé selon l'une quelconque des revendications 1 à 8, dans lequel l'enzyme secondaire est une oxydoréductase, une hydrolase ou une peroxydase, éventuellement dans lequel la peroxydase est la peroxydase de raifort (HRP).

10. Procédé selon l'une quelconque des revendications 1 à 9, dans lequel l'haptène est la biotine, la digoxigénine (DIG), le NP, le BF, le NCA, la BD, le dinitrophényle (DNP), l'oxazole, le pyrazole, le thiazole, le nitroaryle, le benzofurane, un triterpène, une urée, une thiourée, un roténoïde, la coumarine ou un cyclolignane haptène.

11. Procédé selon l'une quelconque des revendications 1 à 10, dans lequel le troisième agent de liaison est un anticorps tertiaire.

12. Procédé selon l'une quelconque des revendications 1 à 11, dans lequel l'enzyme tertiaire est une phosphatase alcaline (PAL).

13. Procédé selon l'une quelconque des revendications 1 à 12, dans lequel le procédé est appliqué dans un procédé de coloration multiplex pour la détection de multiples biomarqueurs cibles.

14. Procédé selon l'une quelconque des revendications 1 à 13, dans lequel le procédé est effectué sur une machine de coloration automatisée.

15. Machine de coloration automatisée comprenant un système prévu pour effectuer un procédé selon l'une quelconque des revendications 1 à 14, dans laquelle la machine de coloration automatisée comprend une mémoire couplée à un processeur, dans laquelle la mémoire stocke des instructions lisibles par ordinateur qui, lorsqu'elles sont exécutées par le processeur, amènent la machine de coloration automatisée à effectuer des opérations pour le procédé selon l'une quelconque des revendications 1 à 14.
